# EUROPEAN PATENT APPLICATION

(11) **EP 4 596 574 A1**
(43) Date of publication of application: **06.08.2025**
(21) Application number: 23870889.5
(22) Date of filing: 27.09.2023
(51) Int. Cl.: C07K 14/605, A61K 38/26, A61P 3/00

(54) **GLP-1/GCG/GIP TRIPLE-RECEPTOR AGONIST AND USE THEREOF**

(30) Priority: 28.09.2022 CN 202211191825
(71) Applicant: Sunshine Lake Pharma Co., Ltd., Dongguan, Guangdong 523000 (CN)
(72) Inventor: JIANG, Peng, Dongguan, Guangdong 523871 (CN); GUO, Linfeng, Dongguan, Guangdong 523871 (CN); ZHOU, Linjun, Dongguan, Guangdong 523871 (CN); SUN, Ningyuan, Dongguan, Guangdong 523871 (CN); XIAO, Lin, Dongguan, Guangdong 523871 (CN); YU, Jianghong, Dongguan, Guangdong 523871 (CN); CHEN, Junying, Dongguan, Guangdong 523871 (CN); LI, Lijia, Dongguan, Guangdong 523871 (CN); LI, Yong, Dongguan, Guangdong 523871 (CN); LI, Ming, Dongguan, Guangdong 523871 (CN); LI, Xiaoping, Dongguan, Guangdong 523871 (CN); LI, Jing, Dongguan, Guangdong 523871 (CN); LI, Wenjia, Dongguan, Guangdong 523871 (CN)
(74) Representative: Hoffmann Eitle
(86) International application number: PCT/CN2023/121877
(87) International publication number: WO 2024/067662

(57) **Abstract**

A triple-receptor agonist. The agonist comprises a polypeptide represented by formula (I), X₁X₂X₃GTX₆TSDYSIX₁₃X₁₄DX₁₆X₁₇X₁₈X₁₉X₂₀X₂₁FX₂₃X₂₄X₂₅LX₂₇X₂₈GGPSSGAPPPS (I), wherein, X₁ is Y or H; X₂ is Aib; X₃ is Q or H; X₆ is an unnatural amino acid; X₁₃ is L or Y; X₁₄ is L or K; X₁₆ is E, K or R; X₁₇ is K, R or I; X₁₈ is A, R or K; X₁₉ is Q or A; X₂₀ is R, K, Q, H or Aib; X₂₁ is D, A or E; X₂₃ is I or V; X₂₄ is E or Q; X₂₅ is W or Y; X₂₇ is I or L; X₂₈ is E or A. The agonist has relatively strong agonistic activity on the three receptors GLP-1, GCG, and GIP, and can be used for treating diseases related to metabolic disorders.

## Description

### FIELD OF THE INVENTION

The present invention belongs to the field of biomedicine technology. Specifically, the present invention relates to a GLP-1/GCG/GIP triple-receptor agonist and use thereof. More specifically, the present invention relates to a compound of formula (I), or a pharmaceutically acceptable salt or solvate, a pharmaceutical composition and use thereof.

### BACKGROUND ART

Diabetes is a metabolic disease closely related to obesity and nonalcoholic steatohepatitis (NASH), and its prevalence and incidence are continuing to increase worldwide. Although there are currently glucagon-like peptide-1 (GLP-1) receptor agonists approved for the treatment of type 2 diabetes mellitus (T2DM), such as dulaglytide, semaglutide, etc., their efficacy is limited due to the comorbidity and heterogeneity of metabolic diseases, especially when type 2 diabetes mellitus is accompanied by multiple concurrent diseases such as obesity. Acting on multiple targets can improve the effectiveness of treating such diseases. However, combined drug administration based on multi-drug combinations, such as combination products, will complicate drug development and clinical research, but the development of single-molecule multi-target drugs can effectively avoid such problems.

Based on the effectiveness and safety of GLP-1 receptor agonist, the current research and development idea is to develop it and other intestinal hormones such as Glucagon (GCG) and Glucose-dependent insulinotropic polypeptide (GIP) into single-molecule multi-target drugs. By acting on multiple targets at the same time, these drugs can balance the side effects and shortcomings of different targets while maintaining stable pharmacokinetic characteristics, ultimately bringing better therapeutic effects than a single target.

### SUMMARY

The present invention aims to solve one of the technical problems existing in the prior art at least to a certain extent. To this end, the present invention provides a GLP-1/GCG/GIP triple-receptor agonist, which can show strong agonist activity on the three receptors of GLP-1, GCG and GIP.

The present invention is accomplished based on the following findings of the inventors:
Glucagon-like peptide-1 (GLP-1) is a polypeptide hormone secreted by intestinal L-cells after food intake. It can stimulate pancreatic *β*-cells to secrete insulin, thereby stabilizing postprandial blood glucose fluctuations. Its blood glucose lowering effect is dependent on glucose concentration. While regulating blood glucose, it greatly reduces the risk of hypoglycemia. In recent years, GLP-1-based drugs, such as liraglutide, dulaglutide and semaglutide, have gradually occupied a very important position in diabetes drugs. GLP-1 drugs can reduce weight while lowering blood glucose. The mechanism is that GLP-1 acts on the gastrointestinal tract to delay gastric emptying and intestinal motility, and acts on the central nervous system to suppress appetite, thereby achieving the purpose of reducing food intake. However, when GLP-1 receptor agonist drugs are used for weight loss, the dosage required is generally high, which is prone to gastrointestinal side effects and poor tolerance.

Glucagon (GCG) is a polypeptide hormone secreted by pancreatic islet α cells. It can promote glycogenolysis and gluconeogenesis, significantly raising blood glucose. It can also activate lipase, promote lipolysis, increase fatty acid oxidation, and increase energy consumption, which contributes to fat loss and weight reduction. Due to its blood glucose-raising effect, although it can be used to treat hypoglycemia, its application in obesity and weight loss is limited, especially in obese people with type II diabetes mellitus.

Glucose-dependent incretin (GIP) is a polypeptide hormone secreted by intestinal K cells. Both GIP and GLP-1 are incretin that promote insulin secretion and lower blood glucose in a blood glucose concentration-dependent manner, and the blood glucose lowering effect mediated by GIP is stronger than that of GLP-1. However, GIP receptor agonists alone can not achieve the goal of improving blood glucose in patients with diabetes because of GIP insensitivity, possibly due to receptor tolerance induced by hyperglycemia.

Studies have found that the causes of metabolic disorder-related diseases (such as diabetes, obesity, and NASH) are complex, while the efficacy of single-target drugs is relatively limited.

Based on this, in one aspect of the present invention, the present invention provides a compound of formula (I) or a pharmaceutically acceptable salt or solvate thereof. According to an embodiment of the present invention, the compound of formula (I) is X₁X₂X₃GTX₆TSDYSIX₁₃X₁₄DX₁₆X₁₇X₁₈X₁₉X₂₀X₂₁FX₂₃X₂₄X₂₅LX₂₇X₂₈GGPSSGAPPPS (I), wherein, X₁ is Y or H; X₂ is Aib; X₃ is Q or H; X₆ is an unnatural amino acid; X₁₃ is L or Y; X₁₄ is L or K; X₁₆ is E, K or R; X₁₇ is K, R or I; X₁₈ is A, R or K; X₁₉ is Q or A; X₂₀ is R, K, Q, H or Aib; X₂₁ is D, A or E; X₂₃ is I or V; X₂₄ is E or Q; X₂₅ is W or Y; X₂₇ is I or L; X₂₈ is E or A; wherein, when X₁₆ is K, X₁₄ is K. The above-mentioned compound of the present invention or pharmaceutically acceptable salt or solvate thereof shows strong agonist activity on the three receptor targets of GLP-1, GCG and GIP. They can effectively control blood glucose and reduce body weight, and can be used to prevent or treat diseases related to metabolic disorders, especially obesity, diabetes, dyslipidemia-related diseases, fatty liver disease, metabolic syndrome and non-alcoholic fatty liver disease.

In other aspect of the present invention, the present invention provides a pharmaceutical composition. According to an embodiment of the present invention, the pharmaceutical composition comprises the aforementioned compound or the pharmaceutically acceptable salt or solvate thereof. The pharmaceutical composition of the present invention can effectively prevent or treat diseases related to metabolic disorders, especially obesity, diabetes, dyslipidemia-related diseases, fatty liver disease, metabolic syndrome and non-alcoholic fatty liver disease.

In another aspect of the present invention, the present invention provides a use of the aforementioned compound or pharmaceutically acceptable salt or solvate thereof or the aforementioned pharmaceutical composition in the preparation of a drug for treating or preventing diseases related to metabolic disorders.

Additional aspects and advantages of the present invention will be given in part in the following description and in part will be obvious from the following description, or will be learned through practice of the present invention.

### Description of the drawings

The above and/or additional aspects and advantages of the present invention will become apparent and easily understood from the description of the embodiments in conjunction with the following drawings, wherein:
Figure 1 is a test result of blood glucose and AUC_{0~90min} of each group in Example 4 of the present invention;
Figure 2 is a test result of blood glucose and AUC_{0~90min} of each group in Example 5 of the present invention;
Figure 3 is a test result of blood glucose of each group in Example 6 of the present invention;
Figure 4 is a test result of glycated hemoglobin of each group in Example 6 of the present invention;
Figure 5 is a test result of the weight change rate of each group in Example 7 of the present invention;
Figure 6 is a test result of the cumulative food intake of each group in Example 7 of the present invention.

### EXAMPLES

The embodiments of the present invention are described in detail below. The embodiments described below are exemplary and are only used to explain the present invention, and are not to be construed as limiting the present invention.

It should be noted that the terms "first" and "second" are only used for descriptive purposes and cannot be understood as indicating or implying relative importance or implicitly indicating the number of the indicated technical features. Therefore, features defined as "first" or "second" may explicitly or implicitly include one or more of such features. Further, in the description of the present invention, unless otherwise specified, "plurality" means two or more.

The term "comprise" or "include" is an open expression, it means comprising the contents disclosed herein, but don't exclude other contents.

The term "optional" or "optionally" refers to that a subsequently described event or circumstance may but need not occur, and that the description includes instances where the event or circumstance occurs and instances in which it does not.

As used herein, the term "solvate" refers to a complex of defined stoichiometry formed between a solute (this refers to the compound according to the present invention or pharmaceutically acceptable salt thereof) and a solvent. The solvent can be water, ethanol or other pharmaceutically acceptable representative small molecule organic substances, such as but not limited to acetic acid or lactic acid. When the above solvent is water, such solvates are generally referred to as hydrates.

Amino acids are referred to herein using the conventional one-letter and three-letter codes for naturally occurring amino acids, as well as the commonly accepted three-letter codes for other α-amino acids, for example, α-aminoisobutyric acid can be represented by either the codes Aib or B. Unless otherwise specified, all amino acid residues in the present invention are preferably in the L-configuration.

Wherein, the structural formula of Aib or B is

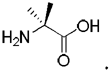

Herein, the terms "(L)-α-Me-(2-F)-Phe" and "αMeF(2F)" are both α-methyl 2-fluorophenylalanine, the structural formula of which is

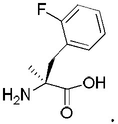

Herein, the sequence structure "-NH₂" refers to the amidation of the -COOH on the C-terminus of the amino acid to -CONH₂. For example, in Y-Aib-QGTX₆TSDYSIL-K ({[2-(2-amino-ethoxy)-ethoxy]-acetyl}-γGlu-CO-C₁₈-CO₂H)DKIAQKAFIEYLLAGGPSSGAPP PS-NH₂, "-NH₂" indicates that the free carboxyl -COOH in the C-terminal serine (S) is amidated to -CONH₂.

Herein, the term "OEG" refers to {[2-(2-amino-ethoxy)-ethoxy]-acetyl, the structural formula of which is shown below:

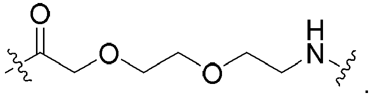

Herein, the term "agonist" refers to a substance (ligand) that activates the mentioned receptor type.

Herein, the term "treating" or "treatment" or "treat" is used to refer to obtaining a desired pharmacological and/or physiological effect. The effect may be prophylactic, in terms of complete or partial prevention of the disease or its symptoms, and/or therapeutic, in terms of partial or complete cure of the disease and/or adverse effects resulting from the disease. Herein, "treatment" or "treating" or "treat" covers diseases in mammals, particularly humans, and includes: (a) preventing the occurrence of a disease or condition in an individual who is susceptible to the disease but has not yet been diagnosed with the disease; (b) inhibiting the disease, such as arresting the progression of the disease; or (c) alleviating the disease, such as alleviating the symptoms associated with the disease. Herein, "treatment" or "treating" or "treat" encompasses any administration of a drug or compound to a subject to treat, cure, alleviate, ameliorate, mitigate or inhibit a disease in the subject, including but not limited to administering a drug containing a compound described herein to a subject in need thereof.

Herein, the term "non-alcoholic fatty liver disease (NAFLD)" generally refers to a clinical pathological syndrome characterized by excessive fat deposition in hepatocytes excluding alcohol and other clear liver-damaging factors, and is an acquired metabolic stress-induced liver injury closely related to insulin resistance and genetic susceptibility, including but not limited to simple fatty liver (SFL), non-alcoholic steatohepatitis (NASH) and its related cirrhosis.

The present invention provides a compound of formula (I) or a pharmaceutically acceptable salt or solvate thereof, a pharmaceutical composition and uses thereof, which will be described in detail below.

### A compound of formula (I) or a pharmaceutically acceptable salt or solvate thereof

In one aspect of the present invention, the present invention provides a compound of formula (I) or a pharmaceutically acceptable salt or solvate thereof. According to an embodiment of the present invention, the compound of formula (I) is X₁X₂X₃GTX₆TSDYSIX₁₃X₁₄DX₁₆X₁₇X₁₈X₁₉X₂₀X₂₁FX₂₃X₂₄X₂₅LX₂₇X₂₈GGPSSGAPPPS (I), wherein, X₁ is Y or H; X₂ is Aib; X₃ is Q or H; X₆ is an unnatural amino acid; X₁₃ is L or Y; X₁₄ is L or K; X₁₆ is E, K or R; X₁₇ is K, R or I; X₁₈ is A, R or K; X₁₉ is Q or A; X₂₀ is R, K, Q, H or Aib; X₂₁ is D, A or E; X₂₃ is I or V; X₂₄ is E or Q; X₂₅ is W or Y; X₂₇ is I or L; X₂₈ is E or A; wherein, when X₁₆ is K, X₁₄ is K. The above-mentioned compound of the present invention or pharmaceutically acceptable salt or solvate thereof (or agonist) shows strong agonist activity on the three receptor targets of GLP-1, GCG and GIP. It can optimize the activity of each receptor and avoid the side effects caused by excessive activity of one or more receptors. It has the advantages of good activity of each receptor and effective control of blood glucose and weight loss. At the same time, it has strong stability *in vivo* and can prolong the drug action time to achieve a frequency of once a week. Therefore, the agonist can be used to prevent or treat diseases related to metabolic disorders, especially obesity, diabetes, dyslipidemia-related diseases, fatty liver disease, metabolic syndrome and non-alcoholic fatty liver disease.

According to an embodiment of the present invention, X₁ is Y; X₂ is Aib; X₃ is Q or H; X₆ is αMeF(2F); X₁₃ is L; X₁₄ is L or K; X₁₆ is E or K; X₁₇ is K or I; X₁₈ is A or R; X₁₉ is Q; X₂₀ is R, Q or Aib; X₂₁ is A or E; X₂₃ is I; X₂₄ is E; X₂₅ is W or Y; X₂₇ is L; X₂₈ is E or A; wherein, when X₁₆ is K, X₁₄ is K. Thereby, the agonist activity on the three receptor targets of GLP-1, GCG and GIP can be further enhanced.

According to an embodiment of the present invention, the compound of formula (I) has at least one of the following structures:
Y-Aib-QGTX₆TSDYSILLDEKAQRDFIEWLLEGGPSSGAPPPS;
Y-Aib-HGTX₆TSDYSILLDEKAQRDFIEWLLAGGPSSGAPPPS;
Y-Aib-HGTX₆TSDYSILLDEIAQKDFIEWLLEGGPSSGAPPPS;
H-Aib-QGTX₆TSDYSILLDEIAQKDFIEWLLEGGPSSGAPPPS;
Y-Aib-QGTX₆TSDYSILLDEKAQQAFIEYLLEGGPSSGAPPPS;
Y-Aib-QGTX₆TSDYSILLDEKAQQDFIEWLIAGGPSSGAPPPS;
Y Aib-QGTX₆TSDYSILLDERAAKEFIEWLLEGGPSSGAPPPS;
Y Aib-QGTX₆TSDYSILLDERAAKEFIEWLIAGGPSSGAPPPS;
Y-Aib-HGTX₆TSDYSILLDEKAQREFIEWLLAGGPSSGAPPPS;
Y-Aib-HGTX₆TSDYSILLDEKAQRAFIEYLLAGGPSSGAPPPS;
Y-Aib-HGTX₆TSDYSILLDEKAQREFIEWLLEGGPSSGAPPPS;
Y-Aib-HGTX₆TSDYSILLDEKRQREFIEWLLAGGPSSGAPPPS;
Y-Aib-QGTX₆TSDYSILLDEKAQQEFIEWLLEGGPSSGAPPPS;
Y-Aib-HGTX₆TSDYSILLDEKAQQEFIEWLLAGGPSSGAPPPS;
Y-Aib-HGTX₆TSDYSILLDEKAQQAFIEYLLAGGPSSGAPPPS;
Y-Aib-QGTX₆TSDYSILLDEKRQQAFIEYLLEGGPSSGAPPPS;
Y-Aib-HGTX₆TSDYSILLDEKAQRAFIEYLLEGGPSSGAPPPS;
Y-Aib-QGTX₆TSDYSILLDEKAQRAFIEYLLEGGPSSGAPPPS;
Y-Aib-HGTX₆TSDYSILLDEKRQREFIEWLLEGGPSSGAPPPS;
Y-Aib-QGTX₆TSDYSILLDEKRARAFIEYLLAGGPSSGAPPPS;
Y-Aib-HGTX₆TSDYSILLDEKRAREFIEWLLEGGPSSGAPPPS;
Y-Aib-QGTX₆TSDYSILLDEKAQQEFIEWLLAGGPSSGAPPPS;
Y-Aib-QGTX₆TSDYSILLDEKAQQEFIEWLIAGGPSSGAPPPS;
Y-Aib-QGTX₆TSDYSILLDEKAQQAFVQWLIAGGPSSGAPPPS;
Y-Aib-QGTX₆TSDYSILLDEKRAQAFIEYLLEGGPSSGAPPPS;
Y-Aib-QGTX₆TSDYSILLDEKAQRAFIEYLLAGGPSSGAPPPS;
Y-Aib-QGTX₆TSDYSILLDEIAQKAFIEYLLAGGPSSGAPPPS;
Y-Aib-QGTX₆TSDYSILLDEIAQKAFIEYLIAGGPSSGAPPPS;
Y-Aib-QGTX₆TSDYSILLDEIAQKAFVQWLIAGGPSSGAPPPS;
Y-Aib-HGTX₆TSDYSILLDEKAQ-Aib-EFIEWLLAGGPSSGAPPPS;
Y-Aib-QGTX₆TSDYSILLDEKAQ-Aib-AFIEYLLAGGPSSGAPPPS;
Y-Aib-HGTX₆TSDYSILLDEKRQ-Aib-EFIEWLLAGGPSSGAPPPS;
Y-Aib-QGTX₆TSDYSILLDEKRQ-Aib-EFIEWLLAGGPSSGAPPPS;
Y-Aib-QGTX₆TSDYSILLDEKRQ-Aib-AFIEYLLAGGPSSGAPPPS;
Y-Aib-QGTX₆TSDYSILKDKIAQQAFIEYLLEGGPSSGAPPPS;
Y-Aib-QGTX₆TSDYSILKDKIAQ-Aib-AFIEYLLEGGPSSGAPPPS;
Y-Aib-HGTX₆TSDYSILLDEKRQQAFIEYLLEGGPSSGAPPPS;
Y-Aib-HGTX₆TSDYSILLDEKRQRAFIEYLLAGGPSSGAPPPS;
Y-Aib-HGTX₆TSDYSILLDEKRQRAFIEYLLEGGPSSGAPPPS;
Y-Aib-HGTX₆TSDYSILLDEKRQKAFIEYLLAGGPSSGAPPPS;
Y-Aib-QGTX₆TSDYSILKDKIAQQAFIEYLLAGGPSSGAPPPS;
Y-Aib-QGTX₆TSDYSILLDEKRQRAFIEYLLEGGPSSGAPPPS;
Y-Aib-QGTX₆TSDYSILLDRIRQKEFIEWLLAGGPSSGAPPPS;
Y-Aib-QGTX₆TSDYSILLDRIRQKAFIEYLLEGGPSSGAPPPS;
Y-Aib-QGTX₆TSDYSILLDRKRQQEFIEWLLAGGPSSGAPPPS;
Y-Aib-QGTX₆TSDYSILLDRKRQQAFIEYLLAGGPSSGAPPPS;
Y-Aib-QGTX₆TSDYSILLDEKKQ-Aib-EFIEWLLAGGPSSGAPPPS;
Y-Aib-QGTX₆TSDYSILLDEKKQKAFIEYLLAGGPSSGAPPPS;
H-Aib-QGTX₆TSDYSILLDEKRQRAFIEYLLAGGPSSGAPPPS;
Y-Aib-QGTX₆TSDYSILLDEKRQRAFIEYLLAGGPSSGAPPPS;
Y-Aib-QGTX₆TSDYSILLDEKRQRAFVEYLLAGGPSSGAPPPS;
Y-Aib-QGTX₆TSDYSIYLDEKAQRAFIEYLLAGGPSSGAPPPS;
Y-Aib-QGTX₆TSDYSIYLDEKAQRAFIEYLLEGGPSSGAPPPS;
Y-Aib-QGTX₆TSDYSILKDKIAQ-Aib-AFIEYLLAGGPSSGAPPPS;
Y-Aib-QGTX₆TSDYSILKDKIAQKAFIEYLLAGGPSSGAPPPS;
Y-Aib-QGTX₆TSDYSILKDKIAQKAFIEYLLEGGPSSGAPPPS;
Y-Aib-QGTX₆TSDYSILKDRIAQRAFIEYLLEGGPSSGAPPPS;
Y-Aib-QGTX₆TSDYSILKDKIRQQAFIEYLLEGGPSSGAPPPS;
Y-Aib-QGTX₆TSDYSILKDKIRQ-Aib-AFIEYLLEGGPSSGAPPPS;
Y-Aib-QGTX₆TSDYSILKDKIRQ-Aib-AFIEYLLAGGPSSGAPPPS;
Y-Aib-QGTX₆TSDYSILKDRIRQQAFIEYLLEGGPSSGAPPPS;
Y-Aib-QGTX₆TSDYSILKDRIRQQAFIEYLLAGGPSSGAPPPS;
Y-Aib-QGTX₆TSDYSILKDRRAQQAFVEYLLAGGPSSGAPPPS;
Y-Aib-QGTX₆TSDYSILKDRRAQQAFVEYLLEGGPSSGAPPPS;
H-Aib-QGTX₆TSDYSILLDEKRQQAFIEYLLEGGPSSGAPPPS;
H-Aib-QGTX₆TSDYSILLDEKRQQAFIEYLLAGGPSSGAPPPS;
H-Aib-HGTX₆TSDYSILLDRKAQHAFIEYLLEGGPSSGAPPPS;
H-Aib-HGTX₆TSDYSILLDRKAQQAFIEYLLAGGPSSGAPPPS;
H-Aib-HGTX₆TSDYSILLDRKAQHAFIEYLLAGGPSSGAPPPS;
H-Aib-QGTX₆TSDYSILLDRKAQHAFIEYLLAGGPSSGAPPPS;
H-Aib-QGTX₆TSDYSILLDRKAQHEFIEWLLEGGPSSGAPPPS;
wherein, X₆ is αMeF(2F).

It should be noted that the "-" between the amino acids in the polypeptide of the above-mentioned compound represents an amide bond. Illustratively, the "-" in "H-Aib-Q" represents an amide bond.

According to an embodiment of the present invention, the compound of formula (I) or a pharmaceutically acceptable salt or solvate thereof further comprises a modification group, and the modification group is connected to at least one amino acid among X₁₄, X₁₇ and X₂₀.

According to an embodiment of the present invention, at least one of X₁₄, X₁₇ and X₂₀ is K, and the modification group is connected to the ε-amino group of the K side chain of at least one of X₁₄, X₁₇ and X₂₀ via an amide bond.

According to an embodiment of the present invention, X₁₄ and/or X₁₇ is K, and the modification group is connected to the ε-amino group of the K side chain of X₁₄ and/or X₁₇ via an amide bond.

According to an embodiment of the present invention, the modification group has the following structure: {[2-(2-amino-ethoxy)-ethoxy]-acetyl}ₐ-(γGlu)_{b}-CO-(CH₂)_{c}-CO₂H, wherein a is any integer from 1 to 3, b is any integer from 1 to 3, and c is any integer from 14 to 20.

In some optional embodiments of the present invention, a is 1. In some optional embodiments of the present invention, a is 2. In some optional embodiments of the present invention, a is 3.

In some optional embodiments of the present invention, b is 1. In some optional embodiments of the present invention, b is 2. In some optional embodiments of the present invention, b is 3.

In some optional embodiments of the present invention, c is any integer between 14 and 20. Exemplarily, c is 14, 15, 16, 17, 18, 19 and 20 or a range value with two values there between as endpoint values, such as 14~18, 16~18 or 16~20.

It should be noted that the "-" in the above-mentioned modification groups represents a chemical bond connecting chemical groups; illustratively, "[2-(2-amino-ethoxy)-ethoxy]-acetyl" is synonymous with "OEG"; "-" in "CO-CH₂" represents a chemical bond connecting chemical groups; a, b and c represent the number of different groups; "Glu" and "E" are synonymous, both referring to glutamic acid; "CO₂H" refers to the COOH group.

According to an embodiment of the present invention, the modification group has at least one of the following structures:

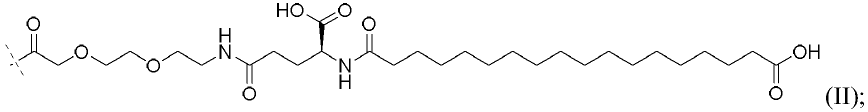

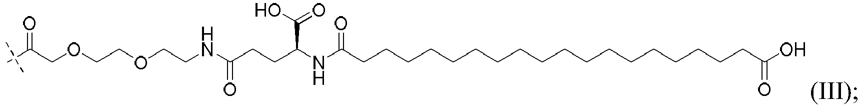

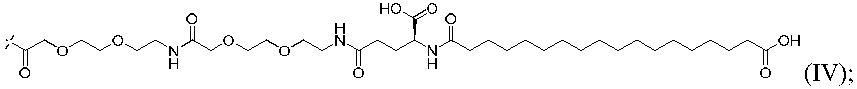

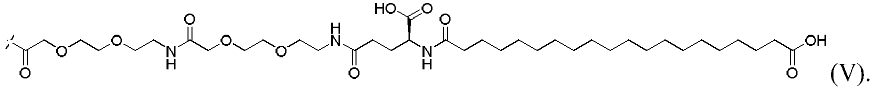

According to an embodiment of the present invention, the C-terminus of the amino acid S at position of 39 of the compound of formula (I) is amidated.

It should be noted that amidation means that the -OH in the -COOH of serine (S) in the compound of formula (I) is replaced by an amino group -NH₂, that is, the -COOH of serine (S) in the compound of formula (I) is amidated to -CONH₂.

According to an embodiment of the present invention, the compound of formula (I) has at least one of the following structures:
Y-Aib-QGTX₆TSDYSILLDE-K({[2-(2-amino-ethoxy)-ethoxy]-acetyl}-γGlu-CO-(CH₂)₁₈-C O₂H) AQRDFIEWLLEGGPSSGAPPPS;
Y-Aib-HGTX₆TSDYSILLDE-K({[2-(2-amino-ethoxy)-ethoxy]-acetyl}-γGlu-CO-(CH₂)₁₈-C O₂H) AQRDFIEWLLAGGPSSGAPPPS;
Y-Aib-HGTX₆TSDYSILLDEIAQ-K({[2-(2-amino-ethoxy)-ethoxy]-acetyl}₂-γGlu-CO-(CH₂ )₁₈-CO₂H) DFIEWLLEGGPSSGAPPPS;
H-Aib-QGTX₆TSDYSILLDEIAQ-K({[2-(2-amino-ethoxy)-ethoxy]-acetyl}₂-γGlu-CO-(CH₂ )₁₈-CO₂H) DFIEWLLEGGPSSGAPPPS;
Y-Aib-QGTX₆TSDYSILLDE-K({[2-(2-amino-ethoxy)-ethoxy]-acetyl}-γGlu-CO-(CH₂)₁₈-C O₂H) AQQAFIEYLLEGGPSSGAPPPS;
Y-Aib-QGTX₆TSDYSILLDE-K({[2-(2-amino-ethoxy)-ethoxy]-acetyl}-γGlu-CO-(CH₂)₁₈-C O₂H) AQQDFIEWLIAGGPSSGAPPPS;
Y-Aib-QGTX₆TSDYSILLDERAA-K({[2-(2-amino-ethoxy)-ethoxy]-acetyl}₂-γGlu-CO-(CH ₂)₁₈-CO₂H) EFIEWLLEGGPSSGAPPPS;
Y-Aib-QGTX₆TSDYSILLDERAA-K({[2-(2-amino-ethoxy)-ethoxy]-acetyl}₂-γGlu-CO-(CH ₂)₁₈-CO₂H) EFIEWLIAGGPSSGAPPPS;
Y-Aib-HGTX₆TSDYSILLDE-K({[2-(2-amino-ethoxy)-ethoxy]-acetyl}-γGlu-CO-(CH₂)₁₆-C O₂H) AQREFIEWLLAGGPSSGAPPPS;
Y-Aib-HGTX₆TSDYSILLDE-K({[2-(2-amino-ethoxy)-ethoxy]-acetyl}-γGlu-CO-(CH₂)₁₆-C O₂H) AQRAFIEYLLAGGPSSGAPPPS;
Y-Aib-HGTX₆TSDYSILLDE-K({[2-(2-amino-ethoxy)-ethoxy]-acetyl}-γGlu-CO-(CH₂)₁₆-C O₂H) AQREFIEWLLEGGPSSGAPPPS;
Y-Aib-HGTX₆TSDYSILLDE-K({[2-(2-amino-ethoxy)-ethoxy]-acetyl}-γGlu-CO-(CH₂)₁₆-C O₂H) RQREFIEWLLAGGPSSGAPPPS;
Y-Aib-QGTX₆TSDYSILLDE-K({[2-(2-amino-ethoxy)-ethoxy]-acetyl}-γGlu-CO-(CH₂)₁₈-C O₂H) AQQEFIEWLLEGGPSSGAPPPS;
Y-Aib-QGTX₆TSDYSILLDE-K({[2-(2-amino-ethoxy)-ethoxy]-acetyl}-γGlu-CO-(CH₂)₁₆-C O₂H) AQQEFIEWLLEGGPSSGAPPPS;
Y-Aib-HGTX₆TSDYSILLDE-K({[2-(2-amino-ethoxy)-ethoxy]-acetyl}-γGlu-CO-(CH₂)₁₆-C O₂H) AQQEFIEWLLAGGPSSGAPPPS;
Y-Aib-HGTX₆TSDYSILLDE-K({[2-(2-amino-ethoxy)-ethoxy]-acetyl}-γGlu-CO-(CH₂)₁₈-C O₂H) AQQAFIEYLLAGGPSSGAPPPS;
Y-Aib-QGTX₆TSDYSILLDE-K({[2-(2-amino-ethoxy)-ethoxy]-acetyl}-γGlu-CO-(CH₂)₁₆-C O₂H) RQQAFIEYLLEGGPSSGAPPPS;
Y-Aib-HGTX₆TSDYSILLDE-K({[2-(2-amino-ethoxy)-ethoxy]-acetyl}-γGlu-CO-(CH₂)₁₈-C O₂H) AQRAFIEYLLAGGPSSGAPPPS;
Y-Aib-HGTX₆TSDYSILLDE-K({[2-(2-amino-ethoxy)-ethoxy]-acetyl}-γGlu-CO-(CH₂)₁₈-C O₂H) AQRAFIEYLLEGGPSSGAPPPS;
Y-Aib-QGTX₆TSDYSILLDE-K({[2-(2-amino-ethoxy)-ethoxy]-acetyl}-γGlu-CO-(CH₂)₁₈-C O₂H) AQRAFIEYLLEGGPSSGAPPPS;
Y-Aib-QGTX₆TSDYSILLDE-K({[2-(2-amino-ethoxy)-ethoxy]-acetyl}-γGlu-CO-(CH₂)₁₈-C O₂H) RQRAFIEYLLAGGPSSGAPPPS;
Y-Aib-HGTX₆TSDYSILLDE-K({[2-(2-amino-ethoxy)-ethoxy]-acetyl}-γGlu-CO-(CH₂)₁₈-C O₂H) RQREFIEWLLEGGPSSGAPPPS;
Y-Aib-QGTX₆TSDYSILLDE-K({[2-(2-amino-ethoxy)-ethoxy]-acetyl}-γGlu-CO-(CH₂)₁₈-C O₂H) RARAFIEYLLAGGPSSGAPPPS;
Y-Aib-HGTX₆TSDYSILLDE-K({[2-(2-amino-ethoxy)-ethoxy]-acetyl}-γGlu-CO-(CH₂)₁₈-C O₂H) RAREFIEWLLEGGPSSGAPPPS;
Y-Aib-QGTX₆TSDYSILLDE-K({[2-(2-amino-ethoxy)-ethoxy]-acetyl}-γGlu-CO-(CH₂)₁₈-C O₂H) AQQEFIEWLLAGGPSSGAPPPS;
Y-Aib-QGTX₆TSDYSILLDE-K({[2-(2-amino-ethoxy)-ethoxy]-acetyl}-γGlu-CO-(CH₂)₁₈-C O₂H) AQQEFIEWLIAGGPSSGAPPPS;
Y-Aib-QGTX₆TSDYSILLDE-K({[2-(2-amino-ethoxy)-ethoxy]-acetyl}-γGlu-CO-(CH₂)₁₈-C O₂H) AQQAFVQWLIAGGPSSGAPPPS;
Y-Aib-HGTX₆TSDYSILLDE-K({[2-(2-amino-ethoxy)-ethoxy]-acetyl}-γGlu-CO-(CH₂)₁₈-C O₂H) RQQAFIEYLLEGGPSSGAPPPS;
Y-Aib-QGTX₆TSDYSILLDE-K({[2-(2-amino-ethoxy)-ethoxy]-acetyl}-γGlu-CO-(CH₂)₁₈-C O₂H) RAQAFIEYLLEGGPSSGAPPPS;
Y-Aib-QGTX₆TSDYSILLDE-K({[2-(2-amino-ethoxy)-ethoxy]-acetyl}-γGlu-CO-(CH₂)₁₈-C O₂H) AQRAFIEYLLAGGPSSGAPPPS;
Y-Aib-HGTX₆TSDYSILLDE-K({[2-(2-amino-ethoxy)-ethoxy]-acetyl}-γGlu-CO-(CH₂)₁₈-C O₂H) RQREFIEWLLAGGPSSGAPPPS;
Y-Aib-QGTX₆TSDYSILLDE-K({[2-(2-amino-ethoxy)-ethoxy]-acetyl}-γGlu-CO-(CH₂)₁₈-C O₂H) RQRAFIEYLLEGGPSSGAPPPS;
Y-Aib-QGTX₆TSDYSILLDEIAQ-K({[2-(2-amino-ethoxy)-ethoxy]-acetyl}₂-γGlu-CO-(CH₂ )₁₈-CO₂H) AFIEYLLAGGPSSGAPPPS;
Y-Aib-QGTX₆TSDYSILLDEIAQ-K({[2-(2-amino-ethoxy)-ethoxy]-acetyl}₂-γGlu-CO-(CH₂ )₁₈-CO₂H) AFIEYLIAGGPSSGAPPPS;
Y-Aib-QGTX₆TSDYSILLDEIAQ-K({[2-(2-amino-ethoxy)-ethoxy]-acetyl}₂-γGlu-CO-(CH₂ )₁₈-CO₂H) AFVQWLIAGGPSSGAPPPS;
Y-Aib-HGTX₆TSDYSILLDE-K({[2-(2-amino-ethoxy)-ethoxy]-acetyl}-γGlu-CO-(CH₂)₁₆-C O₂H) AQ-Aib-EFIEWLLAGGPSSGAPPPS-NH₂;
Y-Aib-QGTX₆TSDYSILLDE-K({[2-(2-amino-ethoxy)-ethoxy]-acetyl}-γGlu-CO-(CH₂)₁₆-C O₂H) AQ-Aib-AFIEYLLAGGPSSGAPPPS-NH₂;
Y-Aib-HGTX₆TSDYSILLDEK({[2-(2-amino-ethoxy)-ethoxy]-acetyl}-γGlu-CO-(CH₂)₁₆-C O₂H) RQ-Aib-EFIEWLLAGGPSSGAPPPS-NH₂;
Y-Aib-QGTX₆TSDYSILLDE-K({[2-(2-amino-ethoxy)-ethoxy]-acetyl}-γGlu-CO-(CH₂)₁₆-C O₂H) RQ-Aib-EFIEWLLAGGPSSGAPPPS-NH₂;
Y-Aib-QGTX₆TSDYSILLDE-K({[2-(2-amino-ethoxy)-ethoxy]-acetyl}-γGlu-CO-(CH₂)₁₆-C O₂H) RQ-Aib-AFIEYLLAGGPSSGAPPPS-NH₂;
Y-Aib-QGTX₆TSDYSIL-K ({[2-(2-amino-ethoxy)-ethoxy]-acetyl}-γGlu-CO-(CH₂)₁₆-CO₂H) DKIAQQAFIEYLLEGGPSSGAPPPS-NH₂;
Y-Aib-QGTX₆TSDYSIL-K ({[2-(2-amino-ethoxy)-ethoxy]-acetyl}-γGlu-CO-(CH₂)₁₆-CO₂H) DKIAQ-Aib-AFIEYLLEGGPSSGAPPPS-NH₂;
Y-Aib-HGTX₆TSDYSILLDE-K({[2-(2-amino-ethoxy)-ethoxy]-acetyl}-γGlu-CO-(CH₂)₁₆-C O₂H) RQQAFIEYLLEGGPSSGAPPPS;
Y-Aib-HGTX₆TSDYSILLDE-K({[2-(2-amino-ethoxy)-ethoxy]-acetyl}-γGlu-CO-(CH₂)₁₆-C O₂H) RQRAFIEYLLAGGPSSGAPPPS;
Y-Aib-HGTX₆TSDYSILLDE-K({[2-(2-amino-ethoxy)-ethoxy]-acetyl}-γGlu-CO-(CH₂)₁₆-C O₂H) RQRAFIEYLLEGGPSSGAPPPS;
Y-Aib-HGTX₆TSDYSILLDE-K({[2-(2-amino-ethoxy)-ethoxy]-acetyl}-γGlu-CO-(CH₂)₁₆-C O₂H) RQKAFIEYLLAGGPSSGAPPPS-NH₂;
Y-Aib-QGTX₆TSDYSIL-K ({[2-(2-amino-ethoxy)-ethoxy]-acetyl}-γGlu-CO-(CH₂)₁₆-CO₂H) DKIAQQAFIEYLLAGGPSSGAPPPS-NH₂;
Y-Aib-QGTX₆TSDYSILLDE-K({[2-(2-amino-ethoxy)-ethoxy]-acetyl}-γGlu-CO-(CH₂)₁₆-C O₂H) RQRAFIEYLLEGGPSSGAPPPS;
Y-Aib-QGTX₆TSDYSILLDRIRQ-K({[2-(2-amino-ethoxy)-ethoxy]-acetyl}-γGlu-CO-(CH₂) ₁₆-CO₂H) EFIEWLLAGGPSSGAPPPS;
Y-Aib-QGTX₆TSDYSILLDRIRQ-K({[2-(2-amino-ethoxy)-ethoxy]-acetyl}-γGlu-CO-(CH₂) ₁₆-CO₂H) AFIEYLLEGGPSSGAPPPS;
Y-Aib-QGTX₆TSDYSILLDR-K({[2-(2-amino-ethoxy)-ethoxy]-acetyl}-γGlu-CO-(CH₂)₁₈-C O₂H) RQQEFIEWLLAGGPSSGAPPPS;
Y-Aib-QGTX₆TSDYSILLDR-K({[2-(2-amino-ethoxy)-ethoxy]-acetyl}-γGlu-CO-(CH₂)₁₈-C O₂H) RQQAFIEYLLAGGPSSGAPPPS;
Y-Aib-QGTX₆TSDYSILLDE-K({[2-(2-amino-ethoxy)-ethoxy]-acetyl}-γGlu-CO-(CH₂)₁₆-C O₂H) KQ-Aib-EFIEWLLAGGPSSGAPPPS-NH₂;
Y-Aib-QGTX₆TSDYSILLDE-K({[2-(2-amino-ethoxy)-ethoxy]-acetyl}-γGlu-CO-(CH₂)₁₆-C O₂H) KQKAFIEYLLAGGPSSGAPPPS-NH₂;
Y-Aib-QGTX₆TSDYSILLDE-K({[2-(2-amino-ethoxy)-ethoxy]-acetyl}-γGlu-CO-(CH₂)₁₈-C O₂H) KQKAFIEYLLAGGPSSGAPPPS-NH₂;
Y-Aib-QGTX₆TSDYSILLDEKKQ-K({[2-(2-amino-ethoxy)-ethoxy]-acetyl}-γGlu-CO-(CH₂ )₁₆-CO₂H) AFIEYLLAGGPSSGAPPPS-NH₂;
H-Aib-QGTX₆TSDYSILLDE-K({[2-(2-amino-ethoxy)-ethoxy]-acetyl}-γGlu-CO-(CH₂)₁₈-C O₂H) RQRAFIEYLLAGGPSSGAPPPS;
Y-Aib-QGTX₆TSDYSILLDE-K({[2-(2-amino-ethoxy)-ethoxy]-acetyl}-γGlu-CO-(CH₂)₁₆-C O₂H) RQRAFIEYLLAGGPSSGAPPPS;
Y-Aib-QGTX₆TSDYSILLDE-K({[2-(2-amino-ethoxy)-ethoxy]-acetyl}-γGlu-CO-(CH₂)₁₆-C O₂H) RQRAFVEYLLAGGPSSGAPPPS;
Y-Aib-QGTX₆TSDYSIYLDE-K({[2-(2-amino-ethoxy)-ethoxy]-acetyl}-γGlu-CO-(CH₂)₁₆-C O₂H) AQRAFIEYLLAGGPSSGAPPPS;
Y-Aib-QGTX₆TSDYSIYLDE-K({[2-(2-amino-ethoxy)-ethoxy]-acetyl}-γGlu-CO-(CH₂)₁₆-C O₂H) AQRAFIEYLLEGGPSSGAPPPS;
Y-Aib-QGTX₆TSDYSIL-K ({[2-(2-amino-ethoxy)-ethoxy]-acetyl}-γGlu-CO-(CH₂)₁₈-CO₂H) DKIAQ-Aib-AFIEYLLAGGPSSGAPPPS-NH₂;
Y-Aib-QGTX₆TSDYSIL-K ({[2-(2-amino-ethoxy)-ethoxy]-acetyl}-γGlu-CO-(CH₂)₁₆-CO₂H) DKIAQ-Aib-AFIEYLLAGGPSSGAPPPS-NH₂;
Y-Aib-QGTX₆TSDYSIL-K ({[2-(2-amino-ethoxy)-ethoxy]-acetyl}-γGlu-CO-(CH₂)₁₆-CO₂H) DKIAQKAFIEYLLAGGPSSGAPPPS-NH₂;
Y-Aib-QGTX₆TSDYSIL-K ({[2-(2-amino-ethoxy)-ethoxy]-acetyl}-γGlu-CO-(CH₂)₁₆-CO₂H) DKIAQKAFIEYLLEGGPSSGAPPPS-NH₂;
Y-Aib-QGTX₆TSDYSIL-K ({[2-(2-amino-ethoxy)-ethoxy]-acetyl}-γGlu-CO-(CH₂)₁₆-CO₂H) DRIAQRAFIEYLLEGGPSSGAPPPS;
Y-Aib-QGTX₆TSDYSIL-K ({[2-(2-amino-ethoxy)-ethoxy]-acetyl}-γGlu-CO-(CH₂)₁₆-CO₂H) DKIRQQAFIEYLLEGGPSSGAPPPS-NH₂;
Y-Aib-QGTX₆TSDYSIL-K ({[2-(2-amino-ethoxy)-ethoxy]-acetyl}-γGlu-CO-(CH₂)₁₆-CO₂H) DKIRQ-Aib-AFIEYLLEGGPSSGAPPPS-NH₂;
Y-Aib-QGTX₆TSDYSIL-K ({[2-(2-amino-ethoxy)-ethoxy]-acetyl}-γGlu-CO-(CH₂)₁₆-CO₂H) DKIRQ-Aib-AFIEYLLAGGPSSGAPPPS-NH₂;
Y-Aib-QGTX₆TSDYSIL-K ({[2-(2-amino-ethoxy)-ethoxy]-acetyl}-γGlu-CO-(CH₂)₁₆-CO₂H) DRIRQQAFIEYLLEGGPSSGAPPPS;
Y-Aib-QGTX₆TSDYSIL-K ({[2-(2-amino-ethoxy)-ethoxy]-acetyl}-γGlu-CO-(CH₂)₁₆-CO₂H) DRIRQQAFIEYLLAGGPSSGAPPPS;
Y-Aib-QGTX₆TSDYSIL-K ({[2-(2-amino-ethoxy)-ethoxy]-acetyl}-γGlu-CO-(CH₂)₁₆-CO₂H) DRRAQQAFVEYLLAGGPSSGAPPPS;
Y-Aib-QGTX₆TSDYSIL-K ({[2-(2-amino-ethoxy)-ethoxy]-acetyl}-γGlu-CO-(CH₂)₁₆-CO₂H) DRRAQQAFVEYLLEGGPSSGAPPPS;
H-Aib-QGTX₆TSDYSILLDE-K({[2-(2-amino-ethoxy)-ethoxy]-acetyl}-γGlu-CO-(CH₂)₁₆-C O₂H) RQQAFIEYLLEGGPSSGAPPPS;
H-Aib-QGTX₆TSDYSILLDE-K({[2-(2-amino-ethoxy)-ethoxy]-acetyl}-γGlu-CO-(CH₂)₁₆-C O₂H) RQQAFIEYLLAGGPSSGAPPPS;
H-Aib-HGTX₆TSDYSILLDR-K({[2-(2-amino-ethoxy)-ethoxy]-acetyl}-γGlu-CO-(CH₂)₁₆-C O₂H) AQHAFIEYLLEGGPSSGAPPPS;
H-Aib-HGTX₆TSDYSILLDR-K({[2-(2-amino-ethoxy)-ethoxy]-acetyl}-γGlu-CO-(CH₂)₁₆-C O₂H) AQQAFIEYLLAGGPSSGAPPPS;
H-Aib-HGTX₆TSDYSILLDR-K({[2-(2-amino-ethoxy)-ethoxy]-acetyl}-γGlu-CO-(CH₂)₁₈-C O₂H) AQHAFIEYLLAGGPSSGAPPPS;
H-Aib-QGTX₆TSDYSILLDR-K({[2-(2-amino-ethoxy)-ethoxy]-acetyl}-γGlu-CO-(CH₂)₁₆-C O₂H) AQHAFIEYLLAGGPSSGAPPPS;
H-Aib-QGTX₆TSDYSILLDR-K({[2-(2-amino-ethoxy)-ethoxy]-acetyl}-γGlu-CO-(CH₂)₁₆-C O₂H) AQHEFIEWLLEGGPSSGAPPPS;
wherein, X₆ is αMeF(2F).

It should be noted that the "-" between the amino acids in the polypeptide of the above compound represents an amide bond; illustratively, the "-" in "H-Aib-Q" represents an amide bond. The "-" in the above-mentioned modification groups represents a chemical bond connecting chemical groups; illustratively, the "-" in "{[2-(2-amino-ethoxy)-ethoxy]-acetyl}-γGlu-CO-C₁₈-CO₂H" represents a chemical bond connecting chemical groups.

### Pharmaceutical Composition

In another aspect of the present invention, the present invention provides a pharmaceutical composition. According to an embodiment of the present invention, the pharmaceutical composition comprises the aforementioned compound or the pharmaceutically acceptable salt or solvate thereof. The pharmaceutical composition of the present invention can effectively prevent or treat diseases related to metabolic disorders, especially obesity, diabetes, dyslipidemia-related diseases, fatty liver disease, metabolic syndrome and non-alcoholic fatty liver disease.

According to an embodiment of the present invention, the pharmaceutical composition further comprises a pharmaceutically acceptable excipient.

### Use

In another aspect of the present invention, the present invention provides use of the aforementioned compound or pharmaceutically acceptable salt or solvate thereof or the aforementioned pharmaceutical composition in the preparation of a drug, wherein the drug is used to prevent or treat diseases related to metabolic disorders.

According to an embodiment of the present invention, the metabolic disorder-related diseases include obesity, diabetes, dyslipidemia-related diseases, fatty liver disease, metabolic syndrome and non-alcoholic fatty liver disease.

### Method for preventing or treating diseases related to metabolic disorders

In another aspect of the present invention, the present invention provides a method for preventing or treating diseases related to metabolic disorders. According to an embodiment of the present invention, the method comprises: administering a pharmaceutically acceptable amount of the aforementioned compound or the pharmaceutically acceptable salt or solvate thereof or the aforementioned pharmaceutical composition to a subject.

According to an embodiment of the present invention, the metabolic disorder-related diseases include obesity, diabetes, dyslipidemia-related diseases, fatty liver disease, metabolic syndrome and non-alcoholic fatty liver disease.

It should be noted that the "pharmaceutically acceptable amount" may vary depending on the mode of administration and the severity of the disease to be treated, and is preferably an effective amount. The selection of a pharmaceutically acceptable amount can be determined by a person of ordinary skill in the art based on various factors (eg, through clinical trials). The factors include, but are not limited to: pharmacokinetic parameters of the active ingredient such as bioavailability, metabolism, half-life, etc.; severity of the disease to be treated, weight of the patient, immune status of the patient, route of administration, etc. For example, several divided doses may be administered daily, or the dose may be proportionally reduced according to the urgent requirements of the therapeutic situation.

The scheme of the present invention will be explained below with reference to embodiments. Those skilled in the art will appreciate that the following embodiments are only used to illustrate the present invention and should not be construed as limiting the scope of the present invention. If no specific techniques or conditions are specified in the examples, the techniques or conditions described in the literature in the art or the product instructions shall be followed. The reagents and instruments used without indicating the manufacturer are all conventional products that can be purchased commercially.

### Example 1: Preparation of polypeptide compounds

1. Human GLP-1, human GCG, human GIP and various polypeptide compounds (triple receptor agonists) were synthesized according to Table 1 below. The specific polypeptide compound structures are shown in Table 1.

**Table 1 Compound structures of triple receptor agonists**

| Name | Sequence | Position of side chain modification | The structure of fatty acid side chains | SEQ ID NO: |
|---|---|---|---|---|
| Human GLP-1 | HAEGTFTSDVSSYLEGQAAKEFIA WLVKGRG | N/A | N/A | 1 |
| Human GCG | HSQGTFTSDYSKYLDSRRAQDFV QWLMNT | N/A | N/A | 2 |
| Human GIP | YAEGTFISDYSIAMDKIHQQDFVN WLLAQKGKKNDWKHNITQ | N/A | N/A | 3 |
| HEC-PT64 | YBQGTXTSDYSILLDEKΛQRDFIE | Position of 17 | -OEG-γGlu-CO-(CH₂)₁₈-CO₂H | 4 |
| | WLLEGGPSSGAPPPS | | | |
| HEC-PT65 | YBHGTXTSDYSILLDEKΛQRDFIE WLLAGGPSSGAPPPS | Position of 17 | -OEG-γGlu-CO-(CH₂)₁₈-CO₂H | 5 |
| HEC-PT66 | YBHGTXTSDYSILLDEIΛQKDFIE WLLEGGPSSGAPPPS | Position of 20 | -OEG-OEG-γGlu-CO-(CH₂)₁₈-CO₂H | 6 |
| HEC-PT67 | HBQGTXTSDYSILLDEIΛQKDFIE WLLEGGPSSGAPPPS | Position of 20 | -OEG-OEG-γGlu-CO-(CH₂)₁₈-CO₂H | 7 |
| HEC-PT68 | YBQGTXTSDYSILLDEKΛQQΛFIE YLLEGGPSSGAPPPS | Position of 17 | -OEG-γGlu-CO-(CH₂)₁₈-CO₂H | 8 |
| HEC-PT69 | YBQGTXTSDYSILLDEKΛQQDFIE WLIAGGPSSGAPPPS | Position of 17 | -OEG-γGlu-CO-(CH₂)₁₈-CO₂H | 9 |
| HEC-PT70 | YBQGTXTSDYSILLDERΛΛKEFIE WLLEGGPSSGAPPPS | Position of 20 | -OEG-OEG-γGlu-CO-(CH₂)₁₈-CO₂H | 10 |
| HEC-PT71 | YBQGTXTSDYSILLDERΛΛKEFIE WLIAGGPSSGAPPPS | Position of 20 | -OEG-OEG-γGlu-CO-(CH₂)₁₈-CO₂H | 11 |
| HEC-PT74 | YBHGTXTSDYSILLDEKAQREFIE WLLAGGPSSGAPPPS | Position of 17 | -OEG-γGlu-CO-(CH₂)₁₆-CO₂H | 12 |
| HEC-PT75 | YBHGTXTSDYSILLDEKΛQRΛFIE YLLAGGPSSGAPPPS | Position of 17 | -OEG-γGlu-CO-(CH₂)₁₆-CO₂H | 13 |
| HEC-PT76 | YBHGTXTSDYSILLDEKΛQREFIE WLLEGGPSSGAPPPS | Position of 17 | -OEG-γGlu-CO-(CH₂)₁₆-CO₂H | 14 |
| HEC-PT77 | YBHGTXTSDYSILLDEKRQREFIE WLLAGGPSSGAPPPS | Position of 17 | -OEG-γGlu-CO-(CH₂)₁₆-CO₂H | 15 |
| HEC-PT78 | YBQGTXTSDYSILLDEKΛQQEFIE WLLEGGPSSGAPPS | Position of 17 | -OEG-γGlu-CO-(CH₂)₁₈-CO₂H | 16 |
| HEC-PT79 | YBQGTXTSDYSILLDEKΛQQEFIE WLLEGGPSSGAPPS | Position of 17 | -OEG-γGlu-CO-(CH₂)₁₆-CO₂H | 17 |
| HEC-PT80 | YBHGTXTSDYSILLDEKΛQQEFIE WLLAGGPSSGAPPPS | Position of 17 | -OEG-γGlu-CO-(CH₂)₁₆-CO₂H | 18 |
| HEC-PT81 | YBHGTXTSDYSILLDEKΛQQΛFIE YLLAGGPSSGAPPPS | Position of 17 | -OEG-γGlu-CO-(CH₂)₁₈-CO₂H | 19 |
| HEC-PT82 | YBQGTXTSDYSILLDEKRQQAFIE YLLEGGPSSGAPPPS | Position of 17 | -OEG-γGlu-CO-(CH₂)₁₆-CO₂H | 20 |
| HEC-PT83 | YBHGTXTSDYSILLDEKΛQRΛFIE YLLAGGPSSGAPPPS | Position of 17 | -OEG-γGlu-CO-(CH₂)₁₈-CO₂H | 21 |
| HEC-PT84 | YBHGTXTSDYSILLDEKΛQRΛFIE YLLEGGPSSGAPPPS | Position of 17 | -OEG-γGlu-CO-(CH₂)₁₈-CO₂H | 22 |
| HEC-PT85 | YBQGTXTSDYSILLDEKΛQRΛFIE YLLEGGPSSGAPPPS | Position of 17 | -OEG-γGlu-CO-(CH₂)₁₈-CO₂H | 23 |
| HEC-PT86 | YBQGTXTSDYSILLDEKRQRΛFIE YLLAGGPSSGAPPPS | Position of 17 | -OEG-γGlu-CO-(CH₂)₁₈-CO₂H | 24 |
| HEC-PT87 | YBHGTXTSDYSILLDEKRQREFIE WLLEGGPSSGAPPPS | Position of 17 | -OEG-γGlu-CO-(CH₂)₁₈-CO₂H | 25 |
| HEC-PT88 | YBQGTXTSDYSILLDEKRΛRΛFIE YLLAGGPSSGAPPPS | Position of 17 | -OEG-γGlu-CO-(CH₂)₁₈-CO₂H | 26 |
| HEC-PT89 | YBHGTXTSDYSILLDEKRΛREFIE WLLEGGPSSGAPPPS | Position of 17 | -OEG-γGlu-CO-(CH₂)₁₈-CO₂H | 27 |
| HEC-PT90 | YBQGTXTSDYSILLDEKΛQQEFIE WLLAGGPSSGAPPS | Position of 17 | -OEG-γGlu-CO-(CH₂)₁₈-CO₂H | 28 |
| HEC-PT91 | YBQGTXTSDYSILLDEKΛQQEFIE WLIAGGPSSGAPPPS | Position of 17 | -OEG-γGlu-CO-(CH₂)₁₈-CO₂H | 29 |
| HEC-PT92 | YBQGTXTSDYSILLDEKAQQAFV QWLIAGGPSSGAPPPS | Position of 17 | -OEG-γGlu-CO-(CH₂)₁₈-CO₂H | 30 |
| HEC-PT93 | YBHGTXTSDYSILLDEKRQQAFIE YLLEGGPSSGAPPPS | Position of 17 | -OEG-γGlu-CO-(CH₂)₁₈-CO₂H | 31 |
| HEC-PT94 | YBQGTXTSDYSILLDEKRΛQΛFIE YLLEGGPSSGAPPPS | Position of 17 | -OEG-γGlu-CO-(CH₂)₁₈-CO₂H | 32 |
| HEC-PT95 | YBQGTXTSDYSILLDEKΛQRΛFIE YLLAGGPSSGAPPPS | Position of 17 | -OEG-γGlu-CO-(CH₂)₁₈-CO₂H | 33 |
| HEC-PT96 | YBHGTXTSDYSILLDEKRQREFIE WLLAGGPSSGAPPS | Position of 17 | -OEG-γGlu-CO-(CH₂)₁₈-CO₂H | 34 |
| HEC-PT97 | YBQGTXTSDYSILLDEKRQRΛFIE YLLEGGPSSGAPPS | Position of 17 | -OEG-γGlu-CO-(CH₂)₁₈-CO₂H | 35 |
| HEC-PT98 | YBQGTXTSDYSILLDEIAQKAFIE YLLAGGPSSGAPPPS | Position of 20 | -OEG-OEG-γGlu-CO-(CH₂)₁₈-CO₂H | 36 |
| HEC-PT99 | YBQGTXTSDYSILLDEIAQKAFIE YLIAGGPSSGAPPPS | Position of 20 | -OEG-OEG-γGlu-CO-(CH₂)₁₈-CO₂H | 37 |
| HEC-PT100 | YBQGTXTSDYSILLDEIAQKAFV QWLIAGGPSSGAPPPS | Position of 20 | -OEG-OEG-γGlu-CO-(CH₂)₁₈-CO₂H | 38 |
| HEC-PT107 | YBHGTXTSDYSILLDEKAQBEFIE WLLAGGPSSGAPPPS-NH2 | Position of 17 | -OEG-γGlu-CO-(CH₂)₁₆-CO₂H | 39 |
| HEC-PT108 | YBQGTXTSDYSILLDEKAQBAFIE YLLAGGPSSGAPPPS-NH2 | Position of 17 | -OEG-γGlu-CO-(CH₂)₁₆-CO₂H | 40 |
| HEC-PT109 | YBHGTXTSDYSILLDEKRQBEFIE WLLAGGPSSGAPPPS-NH2 | Position of 17 | -OEG-γGlu-CO-(CH₂)₁₆-CO₂H | 41 |
| HEC-PT110 | YBQGTXTSDYSILLDEKRQBEFIE WLLAGGPSSGAPPPS-NH2 | Position of 17 | -OEG-γGlu-CO-(CH₂)₁₆-CO₂H | 42 |
| HEC-PT112 | YBQGTXTSDYSILLDEKRQBAFIE YLLAGGPSSGAPPPS-NH2 | Position of 17 | -OEG-γGlu-CO-(CH₂)₁₆-CO₂H | 43 |
| HEC-PT113 | YBQGTXTSDYSILKDKIAQQAFIE YLLEGGPSSGAPPPS-NH2 | Position of 14 | -OEG-γGlu-CO-(CH₂)₁₆-CO₂H | 44 |
| HEC-PT114 | YBQGTXTSDYSILKDKIAQBAFIE YLLEGGPSSGAPPPS-NH2 | Position of 14 | -OEG-γGlu-CO-(CH₂)₁₆-CO₂H | 45 |
| HEC-PT118 | YBHGTXTSDYSILLDEKRQQΛFIE YLLEGGPSSGAPPPS | Position of 17 | -OEG-γGlu-CO-(CH₂)₁₆-CO₂H | 46 |
| HEC-PT119 | YBHGTXTSDYSILLDEKRQRΛFIE YLLAGGPSSGAPPPS | Position of 17 | -OEG-γGlu-CO-(CH₂)₁₆-CO₂H | 47 |
| HEC-PT120 | YBHGTXTSDYSILLDEKRQRΛFIE YLLEGGPSSGAPPPS | Position of 17 | -OEG-γGlu-CO-(CH₂)₁₆-CO₂H | 48 |
| HEC-PT121 | YBHGTXTSDYSILLDEKRQKAFIE YLLAGGPSSGAPPPS-NH2 | Position of 17 | -OEG-γGlu-CO-(CH₂)₁₆-CO₂H | 49 |
| HEC-PT122 | YBQGTXTSDYSILKDKIAQQAFIE YLLAGGPSSGAPPPS-NH2 | Position of 14 | -OEG-γGlu-CO-(CH₂)₁₆-CO₂H | 50 |
| HEC-PT124 | YBQGTXTSDYSILLDEKRQRΛFIE YLLEGGPSSGAPPS | Position of 17 | -OEG-γGlu-CO-(CH₂)₁₆-CO₂H | 51 |
| HEC-PT125 | YBQGTXTSDYSILLDRIRQKEFIE WLLAGGPSSGAPPPS | Position of 20 | -OEG-γGlu-CO-(CH₂)₁₆-CO₂H | 52 |
| HEC-PT126 | YBQGTXTSDYSILLDRIRQKΛFIE YLLEGGPSSGAPPPS | Position of 20 | -OEG-γGlu-CO-(CH₂)₁₆-CO₂H | 53 |
| HEC-PT127 | YBQGTXTSDYSILLDRKRQQEFIE WLLAGGPSSGAPPPS | Position of 17 | -OEG-γGlu-CO-(CH₂)₁₈-CO₂H | 54 |
| HEC-PT128 | YBQGTXTSDYSILLDRKRQQΛFIE | Position of 17 | -OEG-γGlu-CO-(CH₂)₁₈-CO₂H | 55 |
| | YLLAGGPSSGAPPPS | | | |
| HEC-PT129 | YBQGTXTSDYSILLDEKKQBEFIE WLLAGGPSSGAPPPS-NH2 | Position of 17 | -OEG-γGlu-CO-(CH₂)₁₆-CO₂H | 56 |
| HEC-PT130 | YBQGTXTSDYSILLDEKKQKAFIE YLLAGGPSSGAPPPS-NH2 | Position of 17 | -OEG-γGlu-CO-(CH₂)₁₆-CO₂H | 57 |
| HEC-PT131 | YBQGTXTSDYSILLDEKKQKAFIE YLLAGGPSSGAPPPS-NH2 | Position of 17 | -OEG-γGlu-CO-(CH₂)₁₈-CO₂H | 58 |
| HEC-PT132 | YBQGTXTSDYSILLDEKKQKAFIE YLLAGGPSSGAPPPS-NH2 | Position of 20 | -OEG-γGlu-CO-(CH₂)₁₆-CO₂H | 59 |
| HEC-PT133 | HBQGTXTSDYSILLDEKRQRΛFIE YLLAGGPSSGAPPPS | Position of 17 | -OEG-γGlu-CO-(CH₂)₁₈-CO₂H | 60 |
| HEC-PT134 | YBQGTXTSDYSILLDEKRQRΛFIE YLLAGGPSSGAPPPS | Position of 17 | -OEG-γGlu-CO-(CH₂)₁₆-CO₂H | 61 |
| HEC-PT135 | YBQGTXTSDYSILLDEKRQRΛFV EYLLAGGPSSGAPPPS | Position of 17 | -OEG-γGlu-CO-(CH₂)₁₆-CO₂H | 62 |
| HEC-PT136 | YBQGTXTSDYSIYLDEKΛQRΛFIE YLLAGGPSSGAPPPS | Position of 17 | -OEG-γGlu-CO-(CH₂)₁₆-CO₂H | 63 |
| HEC-PT137 | YBQGTXTSDYSIYLDEKΛQRΛFIE YLLEGGPSSGAPPPS | Position of 17 | -OEG-γGlu-CO-(CH₂)₁₆-CO₂H | 64 |
| HEC-PT138 | YBQGTXTSDYSILKDKIAQBAFIE YLLAGGPSSGAPPPS-NH2 | Position of 14 | -OEG-γGlu-CO-(CH₂)₁₈-CO₂H | 65 |
| HEC-PT139 | YBQGTXTSDYSILKDKIAQBAFIE YLLAGGPSSGAPPPS-NH2 | Position of 14 | -OEG-γGlu-CO-(CH₂)₁₆-CO₂H | 66 |
| HEC-PT140 | YBQGTXTSDYSILKDKIAQKAFIE YLLAGGPSSGAPPPS-NH2 | Position of 14 | -OEG-γGlu-CO-(CH₂)₁₆-CO₂H | 67 |
| HEC-PT141 | YBQGTXTSDYSILKDKIAQKAFIE YLLEGGPSSGAPPPS-NH2 | Position of 14 | -OEG-γGlu-CO-(CH₂)₁₆-CO₂H | 68 |
| HEC-PT142 | YBQGTXTSDYSILKDRIΛQRΛFIE YLLEGGPSSGAPPPS | Position of 14 | -OEG-γGlu-CO-(CH₂)₁₆-CO₂H | 69 |
| HEC-PT143 | YBQGTXTSDYSILKDKIRQQAFIE YLLEGGPSSGAPPPS-NH2 | Position of 14 | -OEG-γGlu-CO-(CH₂)₁₆-CO₂H | 70 |
| HEC-PT144 | YBQGTXTSDYSILKDKIRQBAFIE YLLEGGPSSGAPPPS-NH2 | Position of 14 | -OEG-γGlu-CO-(CH₂)₁₆-CO₂H | 71 |
| HEC-PT145 | YBQGTXTSDYSILKDKIRQBAFIE YLLAGGPSSGAPPPS-NH2 | Position of 14 | -OEG-γGlu-CO-(CH₂)₁₆-CO₂H | 72 |
| HEC-PT146 | YBQGTXTSDYSILKDRIRQQΛFIE YLLEGGPSSGAPPPS | Position of 14 | -OEG-γGlu-CO-(CH₂)₁₆-CO₂H | 73 |
| HEC-PT147 | YBQGTXTSDYSILKDRIRQQΛFIE YLLAGGPSSGAPPPS | Position of 14 | -OEG-γGlu-CO-(CH₂)₁₆-CO₂H | 74 |
| HEC-PT148 | YBQGTXTSDYSILKDRRΛQQΛFV EYLLAGGPSSGAPPPS | Position of 14 | -OEG-γGlu-CO-(CH₂)₁₆-CO₂H | 75 |
| HEC-PT149 | YBQGTXTSDYSILKDRRAQQAFV EYLLEGGPSSGAPPPS | Position of 14 | -OEG-γGlu-CO-(CH₂)₁₆-CO₂H | 76 |
| HEC-PT150 | HBQGTXTSDYSILLDEKRQQΛFIE YLLEGGPSSGAPPPS | Position of 17 | -OEG-γGlu-CO-(CH₂)₁₆-CO₂H | 77 |
| HEC-PT151 | HBQGTXTSDYSILLDEKRQQΛFIE YLLAGGPSSGAPPPS | Position of 17 | -OEG-γGlu-CO-(CH₂)₁₆-CO₂H | 78 |
| HEC-PT152 | HBHGTXTSDYSILLDRKΛQHΛFIE YLLEGGPSSGAPPPS | Position of 17 | -OEG-γGlu-CO-(CH₂)₁₆-CO₂H | 79 |
| HEC-PT153 | HBHGTXTSDYSILLDRKΛQQΛFIE YLLAGGPSSGAPPPS | Position of 17 | -OEG-γGlu-CO-(CH₂)₁₆-CO₂H | 80 |
| HEC-PT154 | HBHGTXTSDYSILLDRKΛQHΛFIE YLLAGGPSSGAPPPS | Position of 17 | -OEG-γGlu-CO-(CH₂)₁₈-CO₂H | 81 |
| HEC-PT155 | HBQGTXTSDYSILLDRKAQHAFIE YLLAGGPSSGAPPPS | Position of 17 | -OEG-γGlu-CO-(CH₂)₁₆-CO₂H | 82 |
| HEC-PT156 | HBQGTXTSDYSILLDRKΛQHEFIE WLLEGGPSSGAPPPS | Position of 17 | -OEG-γGlu-CO-(CH₂)₁₈-CO₂H | 83 |
| LY3298176 | YBEGTFTSDYSIBLDKIΛQKΛFVQ WLIAGGPSSGAPPPS | Position of 20 | -OEG-OEG-γGlu-CO-(CH₂)₁₈-CO₂H | 84 |

| | | | | |
|---|---|---|---|---|
| Note: The amino acid code "X" in the sequence column of the above table refers to the unnatural amino acid αMeF(2F) mentioned above. | | | | |

The specific steps for preparing a fatty acid side chain modified polypeptide compound are as follows:
1) The resin was placed in a 150 mL reactor and soaked in 50 mL of dichloromethane (DCM) for 2 hours. The resin was washed with dimethylformamide (DMF) and then drained. This process was repeated four times to drain the resin. The first amino acid (protected) at the Fmoc-C terminal, DCM and N,N-diisopropylethylamine (DIEA) were weighed and added into the reactor, and then the reactor was placed in a shaker at 30 °C for 2 hours. The resin was blocked with methanol solution (methanol: DIEA: DCM = 1:1:2) for half an hour, then washed four times with DMF and dried. A 20% piperidine solution was added to the reactor to remove the Fmoc protecting group. The deprotected resin was washed four times with DMF and then dried.
2) The second amino acid at the C-terminus of Fmoc (protected), 1-hydroxybenzotriazole (HOBT) and N,N-diisopropylcarbodiimide (DIC) were weighed and added into the reactor, and then the reactor was placed in a shaker at 30°C for 1 hour. A small amount of resin was taken and tested by the ninhydrin method. If the resin had color, it meant that the condensation was incomplete and the reaction should be continued. After the reaction was complete, the resin was washed four times with DMF and then dried. A certain amount of 20% piperidine (piperidine/DMF = 1:4) was added to the reactor and the reactor was shaken on a decolorization shaker for 20 minutes to remove the Fmoc protecting group on the resin. The deprotected resin was washed four times with DMF and then dried to detect whether the protection was removed.
3) The amino acids were linked in sequence according to the steps, and one of the Lys was used with a specially protected raw material (Lys attached to the side chain). Boc anhydride was added to protect the N-terminal amino group. The special protecting group of the Lys side chain was removed and the side chain structure was connected according to step 2. The peptide protecting groups were completely removed using a cleavage reagent, and the peptide was cleaved from the resin for purification.

2. The target peptides were separated from impurities by reversed-phase liquid chromatography purification. The collected target peptides were lyophilized into powders and their purity and mass spectrometry were performed for identification. The purity of the target peptides was greater than 95% as determined by HPLC. The molecular weights of the peptides identified by mass spectrometry were consistent with the theoretical molecular weights.

### Example 2: Determination of in vitro cell activity

The peptides, human GLP-1, human GCG and human GIP were respectively acted on HEK293 cells expressing GLP-1R, GCGR and GIPR, and the cAMP produced by the receptor cells was detected using a cAMP detection kit (Cisbio, 62AM6PEC), and the dose-effect curve was established to calculate its EC₅₀. The specific steps were as follows:
1) Prepare Assay buffer: Complete culture medium (DMEM medium + 10% FBS) was taken, and then 4/1000 of 500 mM IBMX stock solution was added, the cAMP-d2 working solution and anti-cAMP-crytate working solution were prepared according to the instructions of the cAMP detection kit;
2) The samples to be tested were diluted into 500 nM stock solution, human control peptides GLP-1, GCG and GIP were diluted into 200 nM stock solution with Assay buffer, and then diluted step by step by adding 20 µL to 80 µL Assay buffer (5-fold dilution), including the stock solution for a total of 8 compound gradients;
3) Preparation of cell suspension: After HEK293-GLP-1R, HEK293-GCGR and HEK293-GIPR cells were taken out of liquid nitrogen, they were immediately placed in a 37°C water bath. After they were completely thawed within 1.5 minutes, the cells were added dropwise into a 15 mL centrifuge tube containing 8 mL of warm culture medium in a clean bench. The tubes were centrifuged at 900 rpm for 5 minutes, the supernatant was discarded, and the cells were resuspended in 1 mL of complete culture medium (by pipetting 15 times). 20 µL of the suspension was immediately taken and mixed with an equal volume of trypan blue. 20 µL was taken to count the number of live cells, and the cells were diluted to 4×10⁵ cells/mL with complete culture medium.
4) The 384-well plate was divided into GLP-1R cell, GCGR cell and GIPR cell areas. The cell suspension was added to the wells of the corresponding area at 5 µL per well using a 12-channel variable-channel adjustable dispenser. Then, the gradient dilutions of the test sample (the polypeptide compound prepared in Example 1) and the positive control (human GLP-1, human GCG or human GIP prepared in Example 1) were added to the 384-well plate of the corresponding cells at 5 µL per well (two replicate wells with the same concentration of samples were added in parallel); negative control: 10 µL assay buffer / each well, 3 wells were set for each 384-well plate, covered with a white sealing film, placed in a 37 °C constant temperature incubator, and taken out after half an hour;
5) Before use, the cAMP-d2 working solution and anti-cAMP-crytate working solution were diluted 20 times with the lysis buffer in the kit, and 5 µL of lysis buffer and 5 µL of diluted anti-cAMP-crytate working solution were added to each well of the negative control. Then, the diluted cAMP-d2 working solution and anti-cAMP-crytate working solution were mixed evenly in a ratio of 1:1, and 10 µL was added to each well of the sample group. The wells were covered with white lids and placed at room temperature in the dark for 1 hour or at 4 °C overnight.
6) The fluorescence values at 665 nm and 620 nm were detected in a multifunctional microplate reader. In GraphPad Prism 6, the signal ratio and sample concentration were nonlinearly fitted using a four-parameter equation to obtain the EC₅₀ value. The specific results are shown in Table 2.

**Table 2: Results of in vitro activity tests of different receptor cells**

| Name | GLP-1R (EC₅₀, nM) | GCGR (EC₅₀, nM) | GIPR (EC₅₀, nM) |
|---|---|---|---|
| Human GLP-1 | 0.0523 | N/A | N/A |
| Human GCG | N/A | 0.0257 | N/A |
| Human GIP | N/A | N/A | 0.1125 |
| HEC-PT64 | 0.6004 | 3.5744 | 0.2485 |
| HEC-PT65 | 0.6243 | 1.3580 | 0.1574 |
| HEC-PT66 | 7.1370 | 12.2514 | 0.2135 |
| HEC-PT67 | 0.6791 | 56.9704 | 0.4022 |
| HEC-PT68 | 0.4153 | 0.7481 | 0.1991 |
| HEC-PT69 | 2.1775 | 30.8362 | 0.4189 |
| HEC-PT71 | 0.3359 | 5.3871 | 0.4638 |
| HEC-PT74 | 0.1035 | 0.3190 | 0.0043 |
| HEC-PT75 | 0.1085 | 0.0672 | 0.0377 |
| HEC-PT77 | 0.0656 | 0.1445 | 0.0227 |
| HEC-PT78 | 0.5316 | 4.5020 | 0.2155 |
| HEC-PT80 | 0.3412 | 1.2684 | 0.0506 |
| HEC-PT81 | 0.4087 | 0.3824 | 0.1321 |
| HEC-PT82 | 0.3599 | 0.6154 | 0.0831 |
| HEC-PT83 | 0.2561 | 0.3111 | 0.1575 |
| HEC-PT84 | 0.2907 | 0.3065 | 0.2320 |
| HEC-PT85 | 0.2548 | 0.6024 | 0.2366 |
| HEC-PT86 | 0.1582 | 0.8065 | 0.1125 |
| HEC-PT88 | 0.1304 | 0.0350 | 0.4854 |
| HEC-PT89 | 0.1718 | 0.0419 | 0.2143 |
| HEC-PT90 | 0.3127 | 7.0747 | 0.1864 |
| HEC-PT92 | 0.1036 | 1.8451 | 0.0779 |
| HEC-PT93 | 1.1743 | 0.3540 | 0.1594 |
| HEC-PT94 | 0.5407 | 0.0442 | 1.3042 |
| HEC-PT95 | 0.1563 | 0.6816 | 0.2373 |
| HEC-PT96 | 0.1580 | 0.1921 | 0.1495 |
| HEC-PT99 | 0.2781 | 12.3120 | 0.1764 |
| HEC-PT100 | 0.0978 | 9.5838 | 0.0518 |
| HEC-PT107 | 0.2672 | 3.5591 | 0.0790 |
| HEC-PT108 | 0.1973 | 0.8635 | 0.0540 |
| HEC-PT109 | 0.1398 | 0.7071 | 0.0520 |
| HEC-PT110 | 0.1155 | 1.3320 | 0.0450 |
| HEC-PT112 | 0.1826 | 0.8708 | 0.0534 |
| HEC-PT113 | 0.1033 | 0.1715 | 0.0188 |
| HEC-PT114 | 0.1198 | 0.1376 | 0.0567 |
| HEC-PT118 | 1.2030 | 0.2212 | 0.1279 |
| HEC-PT119 | 0.1889 | 0.2661 | 0.0484 |
| HEC-PT120 | 0.4061 | 0.1999 | 0.1881 |
| HEC-PT121 | 0.2745 | 0.8905 | 0.0813 |
| HEC-PT122 | 0.0893 | 0.1615 | 0.0276 |
| HEC-PT124 | 0.2279 | 0.3315 | 0.1518 |
| HEC-PT125 | 0.1234 | 0.0269 | 0.0217 |
| HEC-PT126 | 0.8612 | 0.0319 | 0.0589 |
| HEC-PT127 | 0.1270 | 0.0707 | 0.0465 |
| HEC-PT128 | 0.3797 | 0.1280 | 0.0608 |
| HEC-PT129 | 0.1405 | 0.5254 | 0.0439 |
| HEC-PT130 | 0.1698 | 4.8600 | 0.5414 |
| HEC-PT131 | 0.2488 | 8.0700 | 1.1270 |
| HEC-PT134 | 0.1462 | 0.8623 | 0.2074 |
| HEC-PT135 | 0.1904 | 0.9021 | 0.2834 |
| HEC-PT138 | 0.2709 | 0.1747 | 0.0649 |
| HEC-PT139 | 0.1583 | 0.0821 | 0.0359 |
| HEC-PT140 | 0.1522 | 0.2722 | 0.0543 |
| HEC-PT141 | 0.1633 | 0.2162 | 0.0955 |
| HEC-PT142 | 0.1603 | 0.1134 | 0.1423 |
| HEC-PT143 | 0.1397 | 0.1734 | 0.0630 |
| HEC-PT144 | 0.1725 | 0.0366 | 0.0221 |
| HEC-PT145 | 0.1261 | 0.0660 | 0.0316 |
| HEC-PT146 | 0.3646 | 0.1211 | 0.1046 |
| HEC-PT147 | 0.1737 | 0.1698 | 0.0861 |
| HEC-PT148 | 0.0741 | 0.1624 | 0.3121 |
| HEC-PT149 | 0.0868 | 0.1376 | 0.2636 |
| HEC-PT153 | 0.0752 | 0.0126 | 0.0169 |
| HEC-PT155 | 0.0555 | 0.0581 | 0.0509 |
| LY3298176 | 0.8224 | N/A | 0.1202 |

Experimental conclusion: The polypeptide compound of the present invention maintains high agonist activity on GLP-1R, GCGR and GIPR.

### Example 3: Pharmacokinetic studies in rats

The pharmacokinetic study of each polypeptide compound prepared in Example 1 was conducted using male Sprague Dawley rats. There were 3 male rats in each group. The animals were free to drink water and did not fast. The dose was 50 nmol/kg, and a single subcutaneous injection was given (see Table 3 for specific peptide compounds). After administration, plasma was collected on the specified days and time points, and the concentration of each peptide compound in the plasma was detected by LC-MS/MS method. The pharmacokinetic parameters were obtained based on the concentration data, which were used to describe the pharmacokinetic properties of the peptide in rats after subcutaneous injection. Results were as shown in table 3.

**Table 3: Summary of pharmacokinetic parameters of some triple-target peptides in rats**

| Name of polypeptide compound | Tₘₐₓ | Cₘₐₓ | AUCₗₐₛₜ | AUC_{INF} | T_{1/2} |
|---|---|---|---|---|---|
| | (h) | (ng/mL) | (h*ng/mL) | (h*ng/mL) | (h) |
| HEC-PT33 | 7.3±1.2 | 914±125 | 22500±6080 | 22600±6080 | 9±1.4 |
| HEC-PT61 | 16±6.9 | 140±12.9 | 6550±312 | 6660±318 | 19±1.4 |
| HEC-PT72 | 12±2.4 | 989±300 | 39500±6760 | 40300±7010 | 19±1.7 |
| HEC-PT77 | 15±11 | 596±213 | 22000±3870 | 22200±3840 | 11±1.6 |
| HEC-PT82 | 8.7±2.3 | 836±217 | 29100±3030 | 29400±3050 | 17±0.8 |
| HEC-PT93 | 25±2.3 | 794±83.3 | 35400±1990 | 36200±1970 | 19±1.2 |
| HEC-PT96 | 25±2.3 | 840±41.4 | 40300±1930 | 40900±1990 | 17±0.65 |
| HEC-PT113 | 13±9.9 | 714±63.2 | 27700±4110 | 27900±4180 | 11±0.62 |
| HEC-PT119 | 19±9.2 | 974±248 | 43500±10700 | 43800±10700 | 16±1.6 |

| | | | | | |
|---|---|---|---|---|---|
| Note: Tₘₐₓ: Time-to-peak; Cₘₐₓ: peak concentration; AUCₗₐₛₜ: area under the drug-time curve at 0-t; AUC_{INF}: area under the drug-time curve at 0-∞; T_{1/2}: elimination half-life. | | | | | |

Experimental conclusion: Except for PT-61, which is poorly absorbed in rats, other peptides are well absorbed in rats, with higher blood concentrations and longer half-lives.

LY3298176 in the following Examples 4 to 6 is tirzepatide, a new drug for type 2 diabetes mellitus that is commercially available. The Vehicle group refers to the injection of the corresponding solvent, that is, compared with the polypeptide compound drug group, the Vehicle group only does not contain the polypeptide compound.

It should be noted that the significance level annotations in the following Examples 4 ~ 6 are the significance analysis results of each group (LY3298176 group and other polypeptide compound groups) and the Vehicle group respectively.

### Example 4: Glucose tolerance assessment in normal C57BL/6 mice

This example evaluated the effects of HEC-PT74, HEC-PT75, HEC-PT77, HEC-PT78, HEC-PT80, and HEC-PT82 on glucose tolerance in normal C57BL/6 mice.

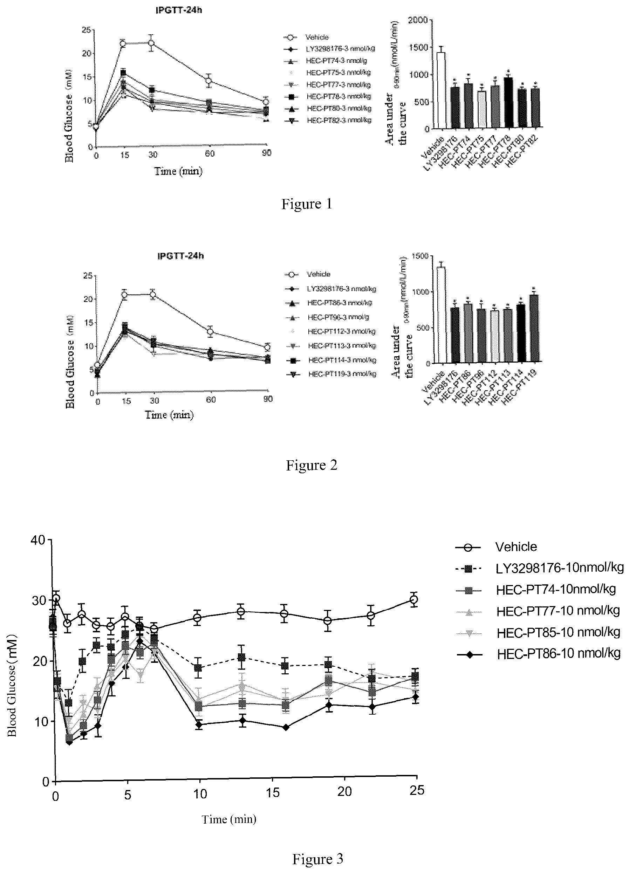

Experimental method: Normal C57BL/6 mice were randomly divided into 8 groups (Vehicle group, LY3298176 group, HEC-PT74 group, HEC-PT75 group, HEC-PT77 group, HEC-PT78 group, HEC-PT80 group, and HEC-PT82 group) according to blood glucose and body weight, with 8 mice in each group. For the LY3298176 group, HEC-PT74 group, HEC-PT75 group, HEC-PT77 group, HEC-PT78 group, HEC-PT80 group and HEC-PT82 group, each group of animals was subcutaneously injected with the corresponding drug, and the dosage was 3 nmol/kg; for the Vehicle group, the corresponding solvent (i.e., PBS solution) was subcutaneously injected. Twelve hours after the single administration, the animals were fasted for 12 hours and had free access to water. Blood was collected from the tail vein to determine the basal blood glucose level of each group of animals, and then 2 g/kg glucose solution was injected intraperitoneally. Blood glucose was tested at 15, 30, 60, and 90 min after glucose administration. The blood glucose concentration-time curve was drawn according to the blood glucose values measured at different time points, and the AUC_{0~90min} of each dose group was calculated as shown in Table 4 and Figure 1.

**Table 4: Effects of single administration of HEC-PT74, HEC-PT75, HEC-PT77, HEC-PT78, HEC-PT80, and HEC-PT82 on glucose tolerance in normal C57 mice 24 h after administration**

| Group | Blood glucose level (mM) | | | | | AUC₀₋₉₀ min |
|---|---|---|---|---|---|---|
| | 0 min | 15 min | 30 min | 60 min | 90 min | |
| Vehicle | 4.5±0.6 | 22.0±2.4 | 22.0±4.9 | 13.8±4.1 | 9.1±2.9 | 1,406.7±293.2 |
| LY3298176 | 4.0±0.8 | 12.6±3.5 | 9.5±3.3 | 7.9±1.4 | 6.5±1.3 | 768.2±171.6^{a} |
| HEC-PT74 | 4.6±1.0 | 14.0±4.3 | 10.0±3.1 | 8.4±2.5 | 7.1±2.3 | 827.3±234.6^{a} |
| HEC-PT75 | 4.0±0.4 | 11.1±3.1 | 8.9±3.1 | 7.4±2.0 | 5.6±0.7 | 682.0±166.7^{a} |
| HEC-PT77 | 4.0±0.5 | 12.6±3.3 | 9.6±3.4 | 8.5±2.7 | 6.9±1.9 | 776.9±227.6^{a} |
| HEC-PT78 | 4.6±0.6 | 15.8±2.6 | 12.0±2.5 | 9.2±1.5 | 7.3±0.7 | 927.1±135.2^{a} |
| HEC-PT80 | 4.6±1.1 | 11.1±2.0 | 9.3±1.7 | 7.2±1.0 | 5.5±0.5 | 707.6±98.4^{a} |
| HEC-PT82 | 3.8±0.6 | 12.4±4.3 | 7.6±1.5 | 6.9±0.8 | 6.6±0.6 | 691.8±116.4^{a} |

| | | | | | | |
|---|---|---|---|---|---|---|
| Note: The lowercase letters on the shoulder in the same column indicate significant differences compared with the Vehicle group (P<0.05). | | | | | | |

Experimental conclusion: 24 hours after a single administration, each administration group can improve the glucose tolerance of normal C57 mice. Among them, HEC-PT75, HEC-PT80 and HEC-PT82 were more effective in improving glucose tolerance than the positive control LY3298176.

### Example 5: Glucose tolerance assessment in normal C57BL/6 mice

This example evaluated the effects of HEC-PT86, HEC-PT96, HEC-PT112, HEC-PT113, HEC-PT114 and HEC-PT119 on glucose tolerance in normal C57BL/6 mice.

Experimental methods: Normal C57BL/6 mice were randomly divided into 8 groups (Vehicle group, LY3298176 group, HEC-PT86 group, HEC-PT96 group, HEC-PT112 group, HEC-PT113 group, HEC-PT114 group, and HEC-PT119 group) according to blood glucose and body weight, with 8 mice in each group. For the LY3298176 group, HEC-PT86 group, HEC-PT96 group, HEC-PT112 group, HEC-PT113 group, HEC-PT114 group, and HEC-PT119 group, each group of animals was subcutaneously injected with the corresponding drug, and the dosage was 3 nmol/kg; for the control group, the corresponding solvent was subcutaneously injected. Twelve hours after the single administration, the animals were fasted for 12 hours and had free access to water. Blood was collected from the tail vein to determine the basal blood glucose level of each group of animals, and then 2 g/kg glucose solution was injected intraperitoneally. Blood glucose was tested at 15, 30, 60, and 90 min after glucose administration. The blood glucose concentration-time curve was drawn according to the blood glucose values measured at different time points, and the AUC_{0~90min} of each dose group was calculated as shown in Table 5 and Figure 2.

**Table 5: Effects of single administration of HEC-PT86, HEC-PT96, HEC-PT112, HEC-PT113, HEC-PT114, and HEC-PT119 on glucose tolerance in normal C57 mice 24 h after administration**

| Group | Blood glucose level (mM) | | | | | AUC_{0-90 min} |
|---|---|---|---|---|---|---|
| | 0 min | 15 min | 30 min | 60 min | 90 min | |
| Vehicle | 6.0±1.2 | 20.8±3.0 | 20.8±2.7 | 12.7±3.1 | 9.1±2.3 | 1,341.9±179.6 |
| LY3298176 | 4.9±1.0 | 13.6±3.1 | 9.9±3.2 | 6.9±0.9 | 7.2±0.9 | 776.9±125.6^{a} |
| HEC-PT86 | 3.9±0.2 | 13.2±1.4 | 10.3±1.9 | 8.8±1.0 | 7.0±0.8 | 827.3±70.3^{a} |
| HEC-PT96 | 4.2±0.6 | 13.0±2.9 | 10.8±3.2 | 7.9±1.6 | 6.2±1.3 | 798.9±146.0^{a} |
| HEC-PT112 | 4.9±1.3 | 11.7±2.6 | 8.6±1.9 | 7.3±0.7 | 6.7±0.6 | 726.2±82.4^{a} |
| HEC-PT113 | 4.6±0.9 | 12.6±1.7 | 8.0±1.0 | 7.9±1.3 | 6.7±0.8 | 743.5±48.5^{a} |
| HEC-PT114 | 4.9±1.0 | 13.5±1.0 | 9.8±0.8 | 7.9±0.8 | 6.3±0.8 | 791.0±55.3^{a} |
| HEC-PT119 | 4.1±0.4 | 14.0±2.2 | 10.8±1.8 | 7.8±0.7 | 6.7±0.7 | 817.4±95.1^{a} |

| | | | | | | |
|---|---|---|---|---|---|---|
| Note: The lowercase letters on the shoulder in the same column indicate significant differences compared with the Vehicle group (P<0.05). | | | | | | |

Experimental conclusion: 24 hours after a single administration, each administration group can improve the glucose tolerance of normal C57 mice. Among them, HEC-PT112 and HEC-PT113 improved glucose tolerance in mice better than the positive control LY3298176.

### Example 6: In vivo efficacy evaluation in db/db mouse model

This example evaluated the effects of HEC-PT74, HEC-PT77, HEC-PT85, and HEC-PT86 on blood glucose in *db*/*db* mice.

Experimental method: 7-8 week old *db*/*db* mice were randomly divided into 6 groups (Vehicle group, LY3298176 group, HEC-PT74 group, HEC-PT77 group, HEC-PT85 group, HEC-PT86 group) according to their blood glucose and body weight values, with 9 mice in each group. For the LY3298176 group, HEC-PT74 group, HEC-PT77 group, HEC-PT85 group and HEC-PT86 group, each group of animals was subcutaneously injected with the corresponding drug, each dose was 10 nmol/kg, and the drug was administered once every three days; for the Vehicle group, the corresponding solvent was subcutaneously injected. Blood glucose was tested before each administration.

Experimental results: HEC-PT74, HEC-PT77, HEC-PT85, and HEC-PT86 groups were able to significantly reduce blood glucose after administration, and blood glucose levels reached the lowest level at about 24 hours, and the effect was slightly better than the positive control LY3298176 at the same dose. Compared with Vehicle, the blood glucose levels of mice in the HEC-PT86 group were significantly reduced after long-term repeated administration and remained stable for a long time. Its hypoglycemic effect was better than that of the LY3298176 group. Specific data are shown in Table 6 and Figures 3-4.

**Table 6: Effects of long-term administration of HEC-PT74, HEC-PT77, HEC-PT85, and HEC-PT86 on blood glucose in db/db mice**

| Group | Random blood glucose (mM) | | | | | | | | | | | | |
|---|---|---|---|---|---|---|---|---|---|---|---|---|---|
| | D0 | 6 h | 24 h | | 48 h | | D3 | | D4 | | D5 | | D6 |
| Vehicle | 25.6±3.7 | 30.3±3.5 | 26.1±4.6 | | 27.6±5.2 | | 25.8±3.3 | | 25.5±4.2 | | 27.1±5.2 | | 25.6±4.2 |
| LY3298176 | 26.1±3.8 | 16.8±5.0 | 13.0±6.7 | | 19.9±5.6 | | 22.4±3.7 | | 22.1±4.0 | | 24.2±3.9 | | 25.3±4.0 |
| HEC-PT74 | 25.5±4.7 | 16.7±5.1 | 7.3±1.6 | | 9.2±2.9 | | 13.5±4.4 | | 20.0±2.9 | | 22.1±3.9 | | 21.1±3.0 |
| HEC-PT77 | 26.6±4.2 | 15.9±5.9 | 8.1±4.5 | | 11.1±6.2 | | 15.8±4.0 | | 17.8±5.9 | | 21.5±5.6 | | 24.1±4.6 |
| HEC-PT85 | 26.2±2.8 | 15.3±5.4 | 10.1±3.7 | | 12.9±4.2 | | 11.8±3.6 | | 17.6±3.1 | | 20.2±5.5 | | 17.3±3.4 |
| HEC-PT86 | 27.3±3.6 | 15.4±4.4 | 6.5±1.4 | | 7.9±2.8 | | 9.2±5.6 | | 16.2±6.5 | | 18.8±5.7 | | 23.0±3.7 |

| Group | Random blood glucose (mM) | | | | | | | | | | | | |
|---|---|---|---|---|---|---|---|---|---|---|---|---|---|
| | D7 | D10 | | D13 | | D16 | | D19 | | D22 | | D25 | |
| Vehicle | 25.0±3.1 | 26.7±3.9 | | 27.6±3.7 | | 27.1±4.6 | | 24.5±6.2 | | 22.3±5.4 | | 29.1±3.6 | |
| LY3298176 | 23.5±2.6 | 18.5±5.1 | | 20.1±5.8 | | 18.6±4.0 | | 18.7±3.8 | | 16.2±4.6 | | 16.6±3.7^{a} | |
| HEC-PT74 | 22.7±2.9 | 12.1±2.1 | | 12.5±2.7 | | 12.1±2.9 | | 16.0±2.3 | | 13.9±3.2 | | 16.3±4.2^{a} | |
| HEC-PT77 | 21.9±3.9 | 13.4±5.4 | | 15.7±5.4 | | 12.7±6.1 | | 15.5±4.6 | | 15.6±6.1 | | 14.2±5.1^{a} | |
| HEC-PT85 | 21.3±3.5 | 11.8±3.8 | | 14.7±4.9 | | 12.8±3.9 | | 13.7±2.5 | | 17.2±4.2 | | 15.7±4.5^{a} | |
| HEC-PT86 | 21.0±4.4 | 9.1±2.3 | | 9.6±3.2 | | 8.4±1.7 | | 12.0±3.3 | | 11.6±3.8 | | 13.1±3.3^{a} | |

| | | | | | | | | | | | | | |
|---|---|---|---|---|---|---|---|---|---|---|---|---|---|
| Note: The lowercase letters on the shoulder in the same column indicate significant differences compared with the Vehicle group (P<0.05). | | | | | | | | | | | | | |

Conclusion: Long-term administration of HEC-PT74, HEC-PT77, HEC-PT85, and HEC-PT86 can significantly improve the blood glucose level of type II diabetes model *db*/*db* mice, and the blood glucose lowering level is not inferior to the positive control LY3298176. Among them, HEC-PT86 has better hypoglycemic effect than LY3298176, and the difference is statistically significant.

### Example 7: Studies in diet-induced obese C57BL/6 mice (DIO)

This example evaluated the effects of long-term repeated administration of HEC-PT77, HEC-PT86 and HEC-PT112 on body weight and food intake in DIO obese mice.

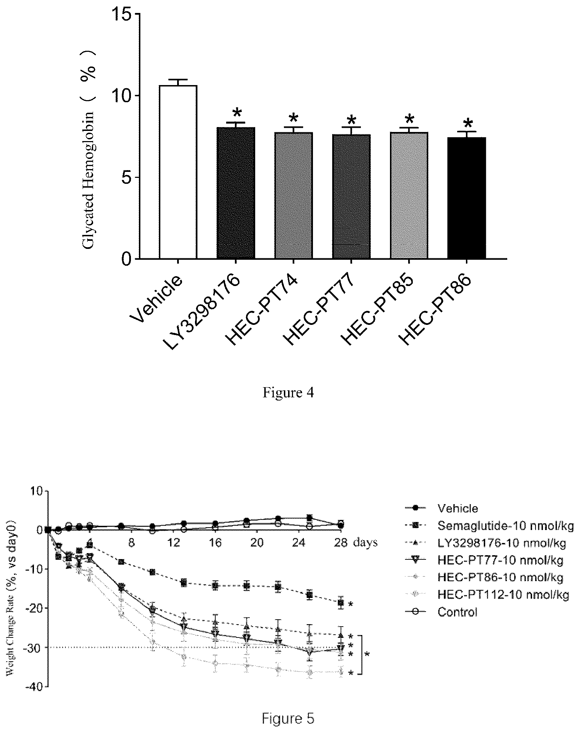

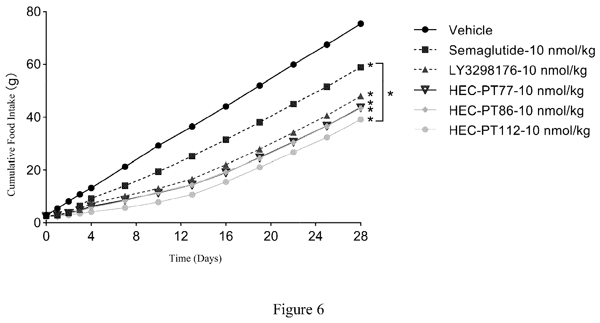

Experimental methods: C57/BL6 mice were randomly divided into a normal group (NFD) and a model group (HFD) at the fifth week of age. The normal group was fed with ordinary maintenance feed, while the other groups were fed with high-fat feed D12492. The body weight and food intake of mice were monitored every 3 weeks. After 16 weeks of feeding, the body weights of mice in the model group and the normal group were (49.0±2.3) g and (31.0±2.0) g, respectively, and the difference between the two groups was statistically significant. The normal group was divided into Control group, and the mice in the successfully modeled group were divided into Vehicle group, Semaglutide group, LY3298176 group, HEC-PT77 group, HEC-PT86 group and HEC-PT112 group, with 10 mice in each group. For the Semaglutide group, LY3298176 group, HEC-PT77 group, HEC-PT86 group and HEC-PT112 group, the corresponding drugs were subcutaneously injected into each group of animals; for the Vehicle group, PBS was subcutaneously injected. The animals were observed for 4 days after the first administration, and then each group was administered once every 3 days, with a dose of 10 nmol/kg for each group. Body weight and food intake were measured before each administration. After 3 weeks of administration, an intraperitoneal glucose tolerance test was performed. The mice were slaughtered and samples were collected 72 h after the last administration. The liver weight was recorded, and the liver pathological conditions and blood biochemical indexes of each group were tested. The test results are shown in Tables 7-10 and Figures 5-6.

Wherein, Semaglutide is commercially available semaglutide.

**Table 7: Effects of long-term administration of HEC-PT77, HEC-PT86 and HEC-PT112 on body weight of DIO mice**

| Group | Weight gain rate (%) | | | | | | |
|---|---|---|---|---|---|---|---|
| | D0 | D1 | D2 | D3 | D4 | D7 | D10 |
| Control | 0.0±0.0 | -0.2±1.5 | 1.2±1.6 | 0.9±1.5 | 1.1±1.8 | 0.9±2.3 | -0.2±2.3 |
| Vehicle | 0.0±0.0 | 0.2±0.5 | 0.4±0.6 | 0.5±0.6 | 0.4±0.9 | 0.4±1.5 | 0.3±1.6 |
| Semaglutide | 0.0±0.0 | -6.8±0.6 | -7.1±1.1 | -5.2±1.3 | -3.8±1.3 | -8.1±1.7 | -10.8±2.2 |
| LY3298176 | 0.0±0.0 | -6.4±0.9 | -8.9±1.3 | -8.7±1.9 | -7.7±2.1 | -15.4±2.9 | -19.6±3.2 |
| HEC-PT77 | 0.0±0.0 | -5.5±0.7 | -7.7±1.9 | -8.4±0.9 | -7.1±3.5 | -14.9±3.8 | -20.9±4.0 |
| HEC-PT86 | 0.0±0.0 | -6.0±0.8 | -8.8±1.4 | -9.9±1.6 | -10.6±2.9 | -17.8±5.8 | -23.5±6.6 |
| HEC-PT112 | 0.0±0.0 | -5.4±0.9 | -8.8±0.8 | -10.6±1.0 | -12.6±1.7 | -21.3±4.0 | -28.6±7.0 |

| Group | Weight gain rate (%) | | | | | | |
|---|---|---|---|---|---|---|---|
| | D13 | D16 | D19 | D22 | D25 | D28 | D31 |
| Control | 0.2±2.2 | 0.8±2.7 | 1.6±2.6 | 1.7±2.1 | 1.0±2.9 | 1.6±2.6 | 3.3±2.8 |
| Vehicle | 1.0±1.8 | 1.2±1.5 | 1.8±1.7 | 2.2±1.8 | 2.4±2.3 | 0.8±1.9 | 1.2±2.4 |
| Semaglutide | -13.5±3.2 | -14.2±3.8 | -14.2±4.1 | -14.5±4.5 | -16.5±4.1 | -18.5±5.1 | -17.4±4.9^{a} |
| LY3298176 | -24.3±6.4 | -23.4±5.1 | -26.0±7.3 | -26.7±7.7 | -27.7±7.0 | -28.2±7.3 | -28.0±6.8^{a} |
| HEC-PT77 | -24.7±5.5 | -26.6±5.6 | -27.7±5.5 | -28.9±6.4 | -31.2±6.9 | -30.4±5.2 | -30.2±4.9^{a} |
| HEC-PT86 | -26.1±7.2 | -27.9±7.6 | -29.2±8.1 | -29.3±7.2 | -30.6±7.0 | -31.2±6.4 | -30.9±6.4^{a} |
| HEC-PT112 | -32.4±7.7 | -34.0±7.1 | -34.5±5.7 | -35.6±5.6 | -36.4±5.1 | -36.2±4.3 | -35.6±4.3^{a} |

| | | | | | | | |
|---|---|---|---|---|---|---|---|
| Note: The lowercase letters on the shoulder in the same column indicate significant differences compared with the Vehicle group (P<0.05). | | | | | | | |

**Table 8: Effects of long-term administration of HEC-PT77, HEC-PT86 and HEC-PT112 on food intake in DIO mice**

| Group | Cumulative food intake (g/pcs) | | | | | | |
|---|---|---|---|---|---|---|---|
| | D0 | D1 | D2 | D3 | D4 | D7 | D10 |
| Control | 3.2 | 6.7 | 10.5 | 14.0 | 17.5 | 28.9 | 39.7 |
| Vehicle | 2.8 | 5.4 | 8.2 | 10.8 | 13.2 | 21.3 | 29.2 |
| Semaglutide | 2.6 | 2.9 | 4.0 | 6.4 | 9.3 | 14.1 | 19.4 |
| LY3298176 | 2.7 | 3.0 | 3.8 | 5.1 | 7.4 | 10.2 | 13.0 |
| HEC-PT77 | 2.4 | 2.8 | 3.6 | 4.7 | 6.3 | 8.7 | 11.4 |
| HEC-PT86 | 2.5 | 2.9 | 3.5 | 4.5 | 5.8 | 8.4 | 11.6 |
| HEC-PT112 | 2.3 | 2.5 | 2.9 | 3.5 | 4.1 | 5.6 | 7.8 |

| Group | Cumulative food intake (g/each) | | | | | | |
|---|---|---|---|---|---|---|---|
| | D13 | D16 | D19 | D22 | D25 | D28 | D30 |
| Control | 51.1 | 62.0 | 72.7 | 83.6 | 94.4 | 106.7 | 132.9 |
| Vehicle | 36.4 | 44.1 | 52.0 | 59.9 | 67.4 | 75.4 | 86.7 |
| Semaglutide | 25.2 | 31.5 | 38.1 | 45.0 | 51.6 | 58.9 | 63.9^{a} |
| LY3298176 | 16.6 | 22.1 | 27.9 | 34.2 | 40.6 | 48.1 | 51.9^{a} |
| HEC-PT77 | 14.4 | 19.1 | 24.7 | 30.6 | 36.7 | 43.7 | 47.5^{a} |
| HEC-PT86 | 14.5 | 19.3 | 24.5 | 30.4 | 36.6 | 43.4 | 47.0^{a} |
| HEC-PT112 | 10.6 | 15.5 | 21.0 | 26.6 | 32.3 | 39.1 | 43.0^{a} |

| | | | | | | | |
|---|---|---|---|---|---|---|---|
| Note: The lowercase letters on the shoulder in the same column indicate significant differences compared with the Vehicle group (P<0.05). | | | | | | | |

**Table 9: Effects of long-term administration of HEC-PT77, HEC-PT86 and HEC-PT112 on body weight and liver index of DIO mice**

| Group | Weight/g | Liver/g | Liver index/% |
|---|---|---|---|
| Control | 29.7±1.8^{a} | 1.1±0.1^{a} | 3.8±0.0^{a} |
| Vehicle | 48.1±2.6 | 2.2±0.4 | 4.6±0.0 |
| Semaglutide | 38.3±2.9^{a} | 1.1±0.1^{a} | 3.0±0.0^{a} |
| LY3298176 | 32.6±3.9^{a} | 1.0±0.1^{a} | 3.0±0.0^{a} |
| HEC-PT77 | 31.5±3.2^{a} | 0.9±0.1^{a} | 2.7±0.0^{a} |
| HEC-PT86 | 32.3±4.4^{a} | 0.9±0.1^{a} | 2.7±0.0^{a} |
| HEC-PT112 | 29.7±2.5^{a} | 0.9±0.1^{a} | 3.0±0.0^{a} |

| | | | |
|---|---|---|---|
| Note: The lowercase letters on the shoulder in the same column indicate significant differences compared with the Vehicle group (P<0.05). | | | |

**Table 10: Effects of long-term administration of HEC-PT77, HEC-PT86 and HEC-PT112 on liver function and blood lipids in DIO mice**

| Group | ALT (U/L) | AST (U/L) | TRIGL (mmol/L) | CHOL (mmol/L) |
|---|---|---|---|---|
| Control | 29.2±4.3^{a} | 123.2±20.7^{a} | 0.8±0.1^{a} | 2.7±0.1^{a} |
| Vehicle | 189.9±37.3 | 244.9±32.0 | 0.9±0.1 | 6.3±0.4 |
| Semaglutide | 45.4±14.6^{a} | 131.2±19.2^{a} | 0.6±0.1^{a} | 4.5±0.6^{a} |
| LY3298176 | 30.9±3.1^{a} | 110.3±17.6^{a} | 0.6±0.1^{a} | 3.3±0.5^{a} |
| HEC-PT77 | 29.1±5.8^{a} | 112.0±20.9^{a} | 0.6±0.1^{a} | 3.2±0.5^{a} |
| HEC-PT86 | 27.8±9.4^{a} | 108.6±26.3^{a} | 0.6±0.1^{a} | 3.5±0.7^{a} |
| HEC-PT112 | 25.9±6.6^{a} | 115.3±16.2^{a} | 0.6±0.1^{a} | 3.1±0.5^{a} |

| | | | | |
|---|---|---|---|---|
| Note: The lowercase letters on the shoulder in the same column indicate significant differences compared with the Vehicle group (P<0.05). | | | | |

Experimental results: After 4 weeks of administration, the weight loss and food intake inhibition effects of HEC-PT77 and HEC-PT86 were comparable to those of the same dose of the positive drug LY3298176, and were superior to the positive drug Semaglutide. The weight loss effect of HEC-PT112 is significantly better than that of LY3298176, and it also has certain advantages in inhibiting food intake. HEC-PT77, HEC-PT86 and HEC-PT112 also significantly improved the liver function and blood lipids of DIO mice, and the effects were similar to those of LY3298176.

Reference throughout this specification to "an embodiment", "some embodiments", "one embodiment", "another example", "an example", "a specific examples", or "some examples" means that a particular feature, structure, material, or characteristic described in connection with the embodiment or example is included in at least one embodiment or example of the present disclosure. Thus, the appearances of the phrases such as "in some embodiments", "in one embodiment", "in an embodiment", "in another example", "in an example", "in a specific examples", or "in some examples" in various places throughout this specification are not necessarily referring to the same embodiment or example of the present disclosure. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

1. A compound of formula (I) or a pharmaceutically acceptable salt or solvate thereof, X₁X₂X₃GTX₆TSDYSIX₁₃X₁₄DX₁₆X₁₇X₁₈X₁₉X₂₀X₂₁FX₂₃X₂₄X₂₅LX₂₇X₂₈GGPSSGAPPPS (I),
wherein X₁ is Y or H;
X₂ is Aib;
X₃ is Q or H;
X₆ is an unnatural amino acid;
X₁₃ is L or Y;
X₁₄ is L or K;
X₁₆ is E, K or R;
X₁₇ is K, R or I;
X₁₈ is A, R or K;
X₁₉ is Q or A;
X₂₀ is R, K, Q, H or Aib;
X₂₁ is D, A or E;
X₂₃ is I or V;
X₂₄ is E or Q;
X₂₅ is W or Y;
X₂₇ is I or L;
X₂₈ is E or A;
wherein, when X₁₆ is K, X₁₄ is K.

2. The compound or a pharmaceutically acceptable salt or solvate thereof of claim 1, wherein X₁ is Y;
X₂ is Aib;
X₃ is Q or H;
X₆ is αMeF(2F);
X₁₃ is L;
X₁₄ is L or K;
X₁₆ is E or K;
X₁₇ is K or I;
X₁₈ is A or R;
X₁₉ is Q;
X₂₀ is R, Q or Aib;
X₂₁ is A or E;
X₂₃ is I;
X₂₄ is E;
X₂₅ is W or Y;
X27 is L;
X₂₈ is E or A;
wherein, when X₁₆ is K, X₁₄ is K.

3. The compound or a pharmaceutically acceptable salt or solvate thereof of claim 1, wherein the compound of formula (I) has at least one of the following structures:
Y-Aib-QGTX₆TSDYSILLDEKAQRDFIEWLLEGGPSSGAPPPS;
Y-Aib-HGTX₆TSDYSILLDEKAQRDFIEWLLAGGPSSGAPPPS;
Y-Aib-HGTX₆TSDYSILLDEIAQKDFIEWLLEGGPSSGAPPPS;
H-Aib-QGTX₆TSDYSILLDEIAQKDFIEWLLEGGPSSGAPPPS;
Y-Aib-QGTX₆TSDYSILLDEKAQQAFIEYLLEGGPSSGAPPPS;
Y-Aib-QGTX₆TSDYSILLDEKAQQDFIEWLIAGGPSSGAPPPS;
Y Aib-QGTX₆TSDYSILLDERAAKEFIEWLLEGGPSSGAPPPS;
Y Aib-QGTX₆TSDYSILLDERAAKEFIEWLIAGGPSSGAPPPS;
Y-Aib-HGTX₆TSDYSILLDEKAQREFIEWLLAGGPSSGAPPPS;
Y-Aib-HGTX₆TSDYSILLDEKAQRAFIEYLLAGGPSSGAPPPS;
Y-Aib-HGTX₆TSDYSILLDEKAQREFIEWLLEGGPSSGAPPPS;
Y-Aib-HGTX₆TSDYSILLDEKRQREFIEWLLAGGPSSGAPPPS;
Y-Aib-QGTX₆TSDYSILLDEKAQQEFIEWLLEGGPSSGAPPPS;
Y-Aib-HGTX₆TSDYSILLDEKAQQEFIEWLLAGGPSSGAPPPS;
Y-Aib-HGTX₆TSDYSILLDEKAQQAFIEYLLAGGPSSGAPPPS;
Y-Aib-QGTX₆TSDYSILLDEKRQQAFIEYLLEGGPSSGAPPPS;
Y-Aib-HGTX₆TSDYSILLDEKAQRAFIEYLLEGGPSSGAPPPS;
Y-Aib-QGTX₆TSDYSILLDEKAQRAFIEYLLEGGPSSGAPPPS;
Y-Aib-HGTX₆TSDYSILLDEKRQREFIEWLLEGGPSSGAPPPS;
Y-Aib-QGTX₆TSDYSILLDEKRARAFIEYLLAGGPSSGAPPPS;
Y Aib-HGTX₆TSDYSILLDEKRAREFIEWLLEGGPSSGAPPPS;
Y-Aib-QGTX₆TSDYSILLDEKAQQEFIEWLLAGGPSSGAPPPS;
Y-Aib-QGTX₆TSDYSILLDEKAQQEFIEWLIAGGPSSGAPPPS;
Y-Aib-QGTX₆TSDYSILLDEKAQQAFVQWLIAGGPSSGAPPPS;
Y-Aib-QGTX₆TSDYSILLDEKRAQAFIEYLLEGGPSSGAPPPS;
Y-Aib-QGTX₆TSDYSILLDEKAQRAFIEYLLAGGPSSGAPPPS;
Y-Aib-QGTX₆TSDYSILLDEIAQKAFIEYLLAGGPSSGAPPPS;
Y-Aib-QGTX₆TSDYSILLDEIAQKAFIEYLIAGGPSSGAPPPS;
Y-Aib-QGTX₆TSDYSILLDEIAQKAFVQWLIAGGPSSGAPPPS;
Y-Aib-HGTX₆TSDYSILLDEKAQ-Aib-EFIEWLLAGGPSSGAPPPS;
Y-Aib-QGTX₆TSDYSILLDEKAQ-Aib-AFIEYLLAGGPSSGAPPPS;
Y-Aib-HGTX₆TSDYSILLDEKRQ-Aib-EFIEWLLAGGPSSGAPPPS;
Y-Aib-QGTX₆TSDYSILLDEKRQ-Aib-EFIEWLLAGGPSSGAPPPS;
Y-Aib-QGTX₆TSDYSILLDEKRQ-Aib-AFIEYLLAGGPSSGAPPPS;
Y-Aib-QGTX₆TSDYSILKDKIAQQAFIEYLLEGGPSSGAPPPS;
Y-Aib-QGTX₆TSDYSILKDKIAQ-Aib-AFIEYLLEGGPSSGAPPPS;
Y-Aib-HGTX₆TSDYSILLDEKRQQAFIEYLLEGGPSSGAPPPS;
Y-Aib-HGTX₆TSDYSILLDEKRQRAFIEYLLAGGPSSGAPPPS;
Y-Aib-HGTX₆TSDYSILLDEKRQRAFIEYLLEGGPSSGAPPPS;
Y-Aib-HGTX₆TSDYSILLDEKRQKAFIEYLLAGGPSSGAPPPS;
Y Aib-QGTX₆TSDYSILKDKIAQQAFIEYLLAGGPSSGAPPPS;
Y-Aib-QGTX₆TSDYSILLDEKRQRAFIEYLLEGGPSSGAPPPS;
Y-Aib-QGTX₆TSDYSILLDRIRQKEFIEWLLAGGPSSGAPPPS;
Y-Aib-QGTX₆TSDYSILLDRIRQKAFIEYLLEGGPSSGAPPPS;
Y-Aib-QGTX₆TSDYSILLDRKRQQEFIEWLLAGGPSSGAPPPS;
Y-Aib-QGTX₆TSDYSILLDRKRQQAFIEYLLAGGPSSGAPPPS;
Y-Aib-QGTX₆TSDYSILLDEKKQ-Aib-EFIEWLLAGGPSSGAPPPS;
Y-Aib-QGTX₆TSDYSILLDEKKQKAFIEYLLAGGPSSGAPPPS;
H-Aib-QGTX₆TSDYSILLDEKRQRAFIEYLLAGGPSSGAPPPS;
Y-Aib-QGTX₆TSDYSILLDEKRQRAFIEYLLAGGPSSGAPPPS;
Y-Aib-QGTX₆TSDYSILLDEKRQRAFVEYLLAGGPSSGAPPPS;
Y-Aib-QGTX₆TSDYSIYLDEKAQRAFIEYLLAGGPSSGAPPPS;
Y-Aib-QGTX₆TSDYSIYLDEKAQRAFIEYLLEGGPSSGAPPPS;
Y-Aib-QGTX₆TSDYSILKDKIAQ-Aib-AFIEYLLAGGPSSGAPPPS;
Y-Aib-QGTX₆TSDYSILKDKIAQKAFIEYLLAGGPSSGAPPPS;
Y-Aib-QGTX₆TSDYSILKDKIAQKAFIEYLLEGGPSSGAPPPS;
Y-Aib-QGTX₆TSDYSILKDRIAQRAFIEYLLEGGPSSGAPPPS;
Y-Aib-QGTX₆TSDYSILKDKIRQQAFIEYLLEGGPSSGAPPPS;
Y-Aib-QGTX₆TSDYSILKDKIRQ-Aib-AFIEYLLEGGPSSGAPPPS;
Y-Aib-QGTX₆TSDYSILKDKIRQ-Aib-AFIEYLLAGGPSSGAPPPS;
Y-Aib-QGTX₆TSDYSILKDRIRQQAFIEYLLEGGPSSGAPPPS;
Y-Aib-QGTX₆TSDYSILKDRIRQQAFIEYLLAGGPSSGAPPPS;
Y-Aib-QGTX₆TSDYSILKDRRAQQAFVEYLLAGGPSSGAPPPS;
Y-Aib-QGTX₆TSDYSILKDRRAQQAFVEYLLEGGPSSGAPPPS;
H-Aib-QGTX₆TSDYSILLDEKRQQAFIEYLLEGGPSSGAPPPS;
H-Aib-QGTX₆TSDYSILLDEKRQQAFIEYLLAGGPSSGAPPPS;
H-Aib-HGTX₆TSDYSILLDRKAQHAFIEYLLEGGPSSGAPPPS;
H-Aib-HGTX₆TSDYSILLDRKAQQAFIEYLLAGGPSSGAPPPS;
H-Aib-HGTX₆TSDYSILLDRKAQHAFIEYLLAGGPSSGAPPPS;
H-Aib-QGTX₆TSDYSILLDRKAQHAFIEYLLAGGPSSGAPPPS;
H-Aib-QGTX₆TSDYSILLDRKAQHEFIEWLLEGGPSSGAPPPS;
wherein, X₆ is αMeF(2F).

4. The compound or a pharmaceutically acceptable salt or solvate thereof of claim 1, further comprising a modification group, wherein the modification group is connected to at least one amino acid among X₁₄, X₁₇ and X₂₀.

5. The compound or a pharmaceutically acceptable salt or solvate thereof of claim 4, wherein at least one of X₁₄, X₁₇ and X₂₀ is K, and the modification group is connected to the ε-amino group of the K side chain of at least one of X₁₄, X₁₇ and X₂₀ via an amide bond;
preferably, X₁₄ and/or X₁₇ is K, and the modification group is connected to the ε-amino group of the K side chain of X₁₄ and/or X₁₇ via an amide bond.

6. The compound or a pharmaceutically acceptable salt or solvate thereof of claim 4, wherein the modification group has the following structure:
{[2-(2-amino-ethoxy)-ethoxy]-acetyl}ₐ-(γGlu)_{b}-CO-(CH₂)_{c}-CO₂H,
wherein, a is any integer from 1 to 3, b is any integer from 1 to 3, and c is any integer from 14 to 20;
preferably, the modification group has at least one of the following structures:

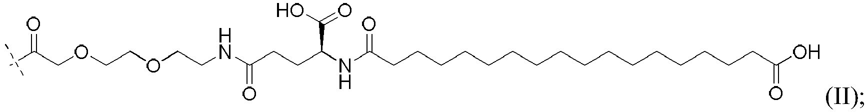

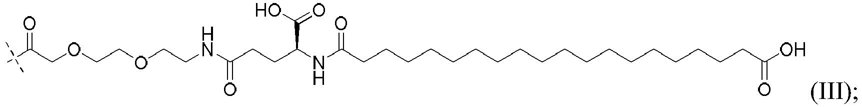

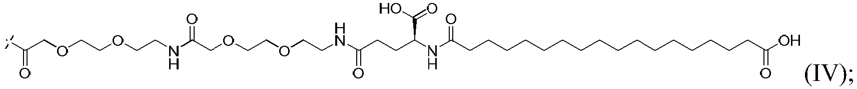

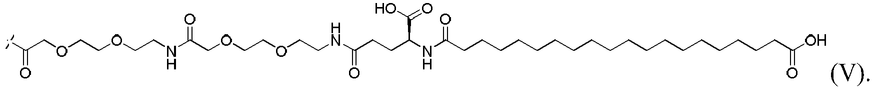

7. The compound or a pharmaceutically acceptable salt or solvate thereof of claim 1, wherein the C-terminus of the amino acid S at position 39 of the compound represented by formula (I) is amidated.

8. The compound or a pharmaceutically acceptable salt or solvate thereof of claim 1, wherein the compound of formula (I) has at least one of the following structures:
Y-Aib-QGTX₆TSDYSILLDE-K({[2-(2-amino-ethoxy)-ethoxy]-acetyl}-γGlu-CO-(CH₂)₁₈-CO₂H) AQRDFIEWLLEGGPSSGAPPPS;
Y-Aib-HGTX₆TSDYSILLDE-K({[2-(2-amino-ethoxy)-ethoxy]-acetyl}-γGlu-CO-(CH₂)₁₈-CO₂H) AQRDFIEWLLAGGPSSGAPPPS;
Y-Aib-HGTX₆TSDYSILLDEIAQ-K({[2-(2-amino-ethoxy)-ethoxy]-acetyl}₂-γGlu-CO-(CH₂)₁₈-CO₂H) DFIEWLLEGGPSSGAPPPS;
H-Aib-QGTX₆TSDYSILLDEIAQ-K({[2-(2-amino-ethoxy)-ethoxy]-acetyl}₂-γGlu-CO-(CH₂)₁₈-CO₂H) DFIEWLLEGGPSSGAPPPS;
Y-Aib-QGTX₆TSDYSILLDE-K({[2-(2-amino-ethoxy)-ethoxy]-acetyl}-γGlu-CO-(CH₂)₁₈-CO₂H) AQQAFIEYLLEGGPSSGAPPPS;
Y-Aib-QGTX₆TSDYSILLDE-K({[2-(2-amino-ethoxy)-ethoxy]-acetyl}-γGlu-CO-(CH₂)₁₈-CO₂H) AQQDFIEWLIAGGPSSGAPPPS;
Y-Aib-QGTX₆TSDYSILLDERAA-K({[2-(2-amino-ethoxy)-ethoxy]-acetyl}₂-γGlu-CO-(CH₂)₁₈-CO₂H) EFIEWLLEGGPSSGAPPPS;
Y-Aib-QGTX₆TSDYSILLDERAA-K({[2-(2-amino-ethoxy)-ethoxy]-acetyl}₂-γGlu-CO-(CH₂)₁₈-CO₂H) EFIEWLIAGGPSSGAPPPS;
Y-Aib-HGTX₆TSDYSILLDE-K({[2-(2-amino-ethoxy)-ethoxy]-acetyl}-γGlu-CO-(CH₂)₁₆-CO₂H) AQREFIEWLLAGGPSSGAPPPS;
Y-Aib-HGTX₆TSDYSILLDE-K({[2-(2-amino-ethoxy)-ethoxy]-acetyl}-γGlu-CO-(CH₂)₁₆-CO₂H) AQRAFIEYLLAGGPSSGAPPPS;
Y-Aib-HGTX₆TSDYSILLDE-K({[2-(2-amino-ethoxy)-ethoxy]-acetyl}-γGlu-CO-(CH₂)₁₆-CO₂H) AQREFIEWLLEGGPSSGAPPPS;
Y-Aib-HGTX₆TSDYSILLDE-K({[2-(2-amino-ethoxy)-ethoxy]-acetyl}-γGlu-CO-(CH₂)₁₆-CO₂H) RQREFIEWLLAGGPSSGAPPPS;
Y-Aib-QGTX₆TSDYSILLDE-K({[2-(2-amino-ethoxy)-ethoxy]-acetyl}-γGlu-CO-(CH₂)₁₈-CO₂H) AQQEFIEWLLEGGPSSGAPPPS;
Y-Aib-QGTX₆TSDYSILLDE-K({[2-(2-amino-ethoxy)-ethoxy]-acetyl}-γGlu-CO-(CH₂)₁₆-CO₂H) AQQEFIEWLLEGGPSSGAPPPS;
Y-Aib-HGTX₆TSDYSILLDE-K({[2-(2-amino-ethoxy)-ethoxy]-acetyl}-γGlu-CO-(CH₂)₁₆-CO₂H) AQQEFIEWLLAGGPSSGAPPPS;
Y-Aib-HGTX₆TSDYSILLDE-K({[2-(2-amino-ethoxy)-ethoxy]-acetyl}-γGlu-CO-(CH₂)₁₈-CO₂H) AQQAFIEYLLAGGPSSGAPPPS;
Y-Aib-QGTX₆TSDYSILLDE-K({[2-(2-amino-ethoxy)-ethoxy]-acetyl}-γGlu-CO-(CH₂)₁₆-CO₂H) RQQAFIEYLLEGGPSSGAPPPS;
Y-Aib-HGTX₆TSDYSILLDE-K({[2-(2-amino-ethoxy)-ethoxy]-acetyl}-γGlu-CO-(CH₂)₁₈-CO₂H) AQRAFIEYLLAGGPSSGAPPPS;
Y-Aib-HGTX₆TSDYSILLDE-K({[2-(2-amino-ethoxy)-ethoxy]-acetyl}-γGlu-CO-(CH₂)₁₈-CO₂H) AQRAFIEYLLEGGPSSGAPPPS;
Y-Aib-QGTX₆TSDYSILLDE-K({[2-(2-amino-ethoxy)-ethoxy]-acetyl}-γGlu-CO-(CH₂)₁₈-CO₂H) AQRAFIEYLLEGGPSSGAPPPS;
Y-Aib-QGTX₆TSDYSILLDE-K({[2-(2-amino-ethoxy)-ethoxy]-acetyl}-γGlu-CO-(CH₂)₁₈-CO₂H) RQRAFIEYLLAGGPSSGAPPPS;
Y-Aib-HGTX₆TSDYSILLDE-K({[2-(2-amino-ethoxy)-ethoxy]-acetyl}-γGlu-CO-(CH₂)₁₈-CO₂H) RQREFIEWLLEGGPSSGAPPPS;
Y-Aib-QGTX₆TSDYSILLDE-K({[2-(2-amino-ethoxy)-ethoxy]-acetyl}-γGlu-CO-(CH₂)₁₈-CO₂H) RARAFIEYLLAGGPSSGAPPPS;
Y-Aib-HGTX₆TSDYSILLDE-K({[2-(2-amino-ethoxy)-ethoxy]-acetyl}-γGlu-CO-(CH₂)₁₈-CO₂H) RAREFIEWLLEGGPSSGAPPPS;
Y-Aib-QGTX₆TSDYSILLDE-K({[2-(2-amino-ethoxy)-ethoxy]-acetyl}-γGlu-CO-(CH₂)₁₈-CO₂H) AQQEFIEWLLAGGPSSGAPPPS;
Y-Aib-QGTX₆TSDYSILLDE-K({[2-(2-amino-ethoxy)-ethoxy]-acetyl}-γGlu-CO-(CH₂)₁₈-CO₂H) AQQEFIEWLIAGGPSSGAPPPS;
Y-Aib-QGTX₆TSDYSILLDE-K({[2-(2-amino-ethoxy)-ethoxy]-acetyl}-γGlu-CO-(CH₂)₁₈-CO₂H) AQQAFVQWLIAGGPSSGAPPPS;
Y-Aib-HGTX₆TSDYSILLDE-K({[2-(2-amino-ethoxy)-ethoxy]-acetyl}-γGlu-CO-(CH₂)₁₈-CO₂H) RQQAFIEYLLEGGPSSGAPPPS;
Y-Aib-QGTX₆TSDYSILLDE-K({[2-(2-amino-ethoxy)-ethoxy]-acetyl}-γGlu-CO-(CH₂)₁₈-CO₂H) RAQAFIEYLLEGGPSSGAPPPS;
Y-Aib-QGTX₆TSDYSILLDE-K({[2-(2-amino-ethoxy)-ethoxy]-acetyl}-γGlu-CO-(CH₂)₁₈-CO₂H) AQRAFIEYLLAGGPSSGAPPPS;
Y-Aib-HGTX₆TSDYSILLDE-K({[2-(2-amino-ethoxy)-ethoxy]-acetyl}-γGlu-CO-(CH₂)₁₈-CO₂H) RQREFIEWLLAGGPSSGAPPPS;
Y-Aib-QGTX₆TSDYSILLDE-K({[2-(2-amino-ethoxy)-ethoxy]-acetyl}-γGlu-CO-(CH₂)₁₈-CO₂H) RQRAFIEYLLEGGPSSGAPPPS;
Y-Aib-QGTX₆TSDYSILLDEIAQ-K({[2-(2-amino-ethoxy)-ethoxy]-acetyl}₂-γGlu-CO-(CH₂)₁₈-CO₂H) AFIEYLLAGGPSSGAPPPS;
Y-Aib-QGTX₆TSDYSILLDEIAQ-K({[2-(2-amino-ethoxy)-ethoxy]-acetyl}₂-γGlu-CO-(CH₂)₁₈-CO₂H) AFIEYLIAGGPSSGAPPPS;
Y-Aib-QGTX₆TSDYSILLDEIAQ-K({[2-(2-amino-ethoxy)-ethoxy]-acetyl}₂-γGlu-CO-(CH₂)₁₈-CO₂H) AFVQWLIAGGPSSGAPPPS;
Y-Aib-HGTX₆TSDYSILLDE-K({[2-(2-amino-ethoxy)-ethoxy]-acetyl}-γGlu-CO-(CH₂)₁₆-CO₂H) AQ-Aib-EFIEWLLAGGPSSGAPPPS-NH₂;
Y-Aib-QGTX₆TSDYSILLDE-K({[2-(2-amino-ethoxy)-ethoxy]-acetyl}-γGlu-CO-(CH₂)₁₆-CO₂H) AQ-Aib-AFIEYLLAGGPSSGAPPPS-NH₂;
Y-Aib-HGTX₆TSDYSILLDEK({[2-(2-amino-ethoxy)-ethoxy]-acetyl}-γGlu-CO-(CH₂)₁₆-CO₂H) RQ-Aib-EFIEWLLAGGPSSGAPPPS-NH₂;
Y-Aib-QGTX₆TSDYSILLDE-K({[2-(2-amino-ethoxy)-ethoxy]-acetyl}-γGlu-CO-(CH₂)₁₆-CO₂H) RQ-Aib-EFIEWLLAGGPSSGAPPPS-NH₂;
Y-Aib-QGTX₆TSDYSILLDE-K({[2-(2-amino-ethoxy)-ethoxy]-acetyl}-γGlu-CO-(CH₂)₁₆-CO₂H) RQ-Aib-AFIEYLLAGGPSSGAPPPS-NH₂;
Y-Aib-QGTX₆TSDYSIL-K({[2-(2-amino-ethoxy)-ethoxy]-acetyl}-γGlu-CO-(CH₂)₁₆-CO₂H) DKIAQQAFIEYLLEGGPSSGAPPPS-NH₂;
Y-Aib-QGTX₆TSDYSIL-K({[2-(2-amino-ethoxy)-ethoxy]-acetyl}-γGlu-CO-(CH₂)₁₆-CO₂H) DKIAQ-Aib-AFIEYLLEGGPSSGAPPPS-NH₂;
Y-Aib-HGTX₆TSDYSILLDE-K({[2-(2-amino-ethoxy)-ethoxy]-acetyl}-γGlu-CO-(CH₂)₁₆-CO₂H) RQQAFIEYLLEGGPSSGAPPPS;
Y-Aib-HGTX₆TSDYSILLDE-K({[2-(2-amino-ethoxy)-ethoxy]-acetyl}-γGlu-CO-(CH₂)₁₆-CO₂H) RQRAFIEYLLAGGPSSGAPPPS;
Y-Aib-HGTX₆TSDYSILLDE-K({[2-(2-amino-ethoxy)-ethoxy]-acetyl}-γGlu-CO-(CH₂)₁₆-CO₂H) RQRAFIEYLLEGGPSSGAPPPS;
Y-Aib-HGTX₆TSDYSILLDE-K({[2-(2-amino-ethoxy)-ethoxy]-acetyl}-γGlu-CO-(CH₂)₁₆-CO₂H) RQKAFIEYLLAGGPSSGAPPPS-NH₂;
Y-Aib-QGTX₆TSDYSIL-K({[2-(2-amino-ethoxy)-ethoxy]-acetyl}-γGlu-CO-(CH₂)₁₆-CO₂H) DKIAQQAFIEYLLAGGPSSGAPPPS-NH₂;
Y-Aib-QGTX₆TSDYSILLDE-K({[2-(2-amino-ethoxy)-ethoxy]-acetyl}-γGlu-CO-(CH₂)₁₆-CO₂H) RQRAFIEYLLEGGPSSGAPPPS;
Y-Aib-QGTX₆TSDYSILLDRIRQ-K({[2-(2-amino-ethoxy)-ethoxy]-acetyl}-γGlu-CO-(CH₂)₁₆-CO₂H) EFIEWLLAGGPSSGAPPPS;
Y-Aib-QGTX₆ TSDYSILLDRIRQ-K({[2-(2-amino-ethoxy)-ethoxy]-acetyl}-γGlu-CO-(CH₂)₁₆-CO₂H) AFIEYLLEGGPSSGAPPPS;
Y-Aib-QGTX₆TSDYSILLDR-K({[2-(2-amino-ethoxy)-ethoxy]-acetyl}-γGlu-CO-(CH₂)₁₈-CO₂H) RQQEFIEWLLAGGPSSGAPPPS;
Y-Aib-QGTX₆TSDYSILLDR-K({[2-(2-amino-ethoxy)-ethoxy]-acetyl}-γGlu-CO-(CH₂)₁₈-CO₂H) RQQAFIEYLLAGGPSSGAPPPS;
Y-Aib-QGTX₆TSDYSILLDE-K({[2-(2-amino-ethoxy)-ethoxy]-acetyl}-γGlu-CO-(CH₂)₁₆-CO₂H) KQ-Aib-EFIEWLLAGGPSSGAPPPS-NH₂;
Y-Aib-QGTX₆TSDYSILLDE-K({[2-(2-amino-ethoxy)-ethoxy]-acetyl}-γGlu-CO-(CH₂)₁₆-CO₂H) KQKAFEEYLLAGGPSSGAPPPS-NH₂;
Y-Aib-QGTX₆TSDYSILLDE-K({[2-(2-amino-ethoxy)-ethoxy]-acetyl}-γGlu-CO-(CH₂)₁₈-CO₂H) KQKAFEEYLLAGGPSSGAPPPS-NH₂;
Y-Aib-QGTX₆TSDYSILLDEKKQ-K({[2-(2-amino-ethoxy)-ethoxy]-acetyl}-γGlu-CO-(CH₂)₁₆-CO₂H) AFIEYLLAGGPSSGAPPPS-NH₂;
H-Aib-QGTX₆TSDYSILLDE-K({[2-(2-amino-ethoxy)-ethoxy]-acetyl}-γGlu-CO-(CH₂)₁₈-CO₂H) RQRAFIEYLLAGGPSSGAPPPS;
Y-Aib-QGTX₆TSDYSILLDE-K({[2-(2-amino-ethoxy)-ethoxy]-acetyl}-γGlu-CO-(CH₂)₁₆-CO₂H) RQRAFIEYLLAGGPSSGAPPPS;
Y-Aib-QGTX₆TSDYSILLDE-K({[2-(2-amino-ethoxy)-ethoxy]-acetyl}-γGlu-CO-(CH₂)₁₆-CO₂H) RQRAFVEYLLAGGPSSGAPPPS;
Y-Aib-QGTX₆TSDYSIYLDE-K({[2-(2-amino-ethoxy)-ethoxy]-acetyl}-γGlu-CO-(CH₂)₁₆-CO₂H) AQRAFIEYLLAGGPSSGAPPPS;
Y-Aib-QGTX₆TSDYSIYLDE-K({[2-(2-amino-ethoxy)-ethoxy]-acetyl}-γGlu-CO-(CH₂)₁₆-CO₂H) AQRAFIEYLLEGGPSSGAPPPS;
Y-Aib-QGTX₆TSDYSIL-K({[2-(2-amino-ethoxy)-ethoxy]-acetyl}-γGlu-CO-(CH₂)₁₈-CO₂H) DKIAQ-Aib-AFIEYLLAGGPSSGAPPPS-NH₂;
Y-Aib-QGTX₆TSDYSIL-K({ [2-(2-amino-ethoxy)-ethoxy]-acetyl } -γGlu-CO-(CH₂)₁₆-CO₂H) DKIAQ-Aib-AFIEYLLAGGPSSGAPPPS-NH₂;
Y-Aib-QGTX₆TSDYSIL-K({ [2-(2-amino-ethoxy)-ethoxy]-acetyl } -γGlu-CO-(CH₂)₁₆-CO₂H) DKIAQKAFIEYLLAGGPSSGAPPPS-NH₂;
Y-Aib-QGTX₆TSDYSIL-K({[2-(2-amino-ethoxy)-ethoxy]-acetyl}-γGlu-CO-(CH₂)₁₆-CO₂H) DKIAQKAFIEYLLEGGPSSGAPPPS-NH₂;
Y-Aib-QGTX₆TSDYSIL-K({[2-(2-amino-ethoxy)-ethoxy]-acetyl}-γGlu-CO-(CH₂)₁₆-CO₂H) DRIAQRAFIEYLLEGGPSSGAPPPS;
Y-Aib-QGTX₆TSDYSIL-K({ [2-(2-amino-ethoxy)-ethoxy]-acetyl } -γGlu-CO-(CH₂)₁₆-CO₂H) DKIRQQAFIEYLLEGGPSSGAPPPS-NH₂;
Y-Aib-QGTX₆TSDYSIL-K({[2-(2-amino-ethoxy)-ethoxy]-acetyl}-γGlu-CO-(CH₂)₁₆-CO₂H) DKIRQ-Aib-AFIEYLLEGGPSSGAPPPS-NH₂;
Y-Aib-QGTX₆TSDYSIL-K({ [2-(2-amino-ethoxy)-ethoxy]-acetyl } -γGlu-CO-(CH₂)₁₆-CO₂H) DKIRQ-Aib-AFIEYLLAGGPSSGAPPPS-NH₂;
Y-Aib-QGTX₆TSDYSIL-K({[2-(2-amino-ethoxy)-ethoxy]-acetyl}-γGlu-CO-(CH₂)₁₆-CO₂H) DRIRQQAFIEYLLEGGPSSGAPPPS;
Y-Aib-QGTX₆TSDYSIL-K({[2-(2-amino-ethoxy)-ethoxy]-acetyl}-γGlu-CO-(CH₂)₁₆-CO₂H) DRIRQQAFIEYLLAGGPSSGAPPPS;
Y-Aib-QGTX₆TSDYSIL-K({[2-(2-amino-ethoxy)-ethoxy]-acetyl } -γGlu-CO-(CH₂)₁₆-CO₂H) DRRAQQAFVEYLLAGGPSSGAPPPS;
Y-Aib-QGTX₆TSDYSIL-K({[2-(2-amino-ethoxy)-ethoxy]-acetyl}-γGlu-CO-(CH₂)₁₆-CO₂H) DRRAQQAFVEYLLEGGPSSGAPPPS;
H-Aib-QGTX₆TSDYSILLDE-K({[2-(2-amino-ethoxy)-ethoxy]-acetyl}-γGlu-CO-(CH₂)₁₆-CO₂H) RQQAFIEYLLEGGPSSGAPPPS;
H-Aib-QGTX₆TSDYSILLDE-K({[2-(2-amino-ethoxy)-ethoxy]-acetyl}-γGlu-CO-(CH₂)₁₆-CO₂H) RQQAFIEYLLAGGPSSGAPPPS;
H-Aib-HGTX₆TSDYSILLDR-K({[2-(2-amino-ethoxy)-ethoxy]-acetyl}-γGlu-CO-(CH₂)₁₆-CO₂H) AQHAFIEYLLEGGPSSGAPPPS;
H-Aib-HGTX₆TSDYSILLDR-K({[2-(2-amino-ethoxy)-ethoxy]-acetyl}-γGlu-CO-(CH₂)₁₆-CO₂H) AQQAFIEYLLAGGPSSGAPPPS;
H-Aib-HGTX₆TSDYSILLDR-K({[2-(2-amino-ethoxy)-ethoxy]-acetyl}-γGlu-CO-(CH₂)₁₈-C0₂H) AQHAFIEYLLAGGPSSGAPPPS;
H-Aib-QGTX₆TSDYSILLDR-K({[2-(2-amino-ethoxy)-ethoxy]-acetyl}-γGlu-CO-(CH₂)₁₆-CO₂H) AQHAFIEYLLAGGPSSGAPPPS;
H-Aib-QGTX₆TSDYSILLDR-K({[2-(2-amino-ethoxy)-ethoxy]-acetyl}-γGlu-CO-(CH_{Z})₁₆-CO₂H) AQHEFIEWLLEGGPSSGAPPPS;
wherein, X₆ is αMeF(2F).

9. A pharmaceutical composition, comprising the compound or a pharmaceutically acceptable salt or solvate thereof of any one of claims 1 to 8.

10. The pharmaceutical composition according to claim 9, further comprising a pharmaceutically acceptable excipient.

11. Use of the compound or the pharmaceutically acceptable salt or solvate thereof of any one of claims 1 to 8 or the pharmaceutical composition of any one of claims 9 to 10 in the manufacture of a medicament for treating or preventing diseases related to metabolic disorders.

12. The use of claim 11, wherein the metabolic disorder-related diseases include at least one of obesity, diabetes, dyslipidemia-related diseases, fatty liver disease, metabolic syndrome and non-alcoholic fatty liver disease.

13. A method of treating or preventing a disease related to a metabolic disorder, comprising administering to the individual the compound or the pharmaceutically acceptable salt or solvate thereof of any one of claims 1 to 8 or the pharmaceutical composition of any one of claims 9 to 10;
wherein the metabolic disorder-related diseases include at least one of obesity, diabetes, dyslipidemia-related diseases, fatty liver disease, metabolic syndrome and non-alcoholic fatty liver disease.

14. The compound or the pharmaceutically acceptable salt or solvate thereof of any one of claims 1 to 8 or the pharmaceutical composition of any one of claims 9 to 10 for use in treating or preventing metabolic disorder-related diseases in the individual;
wherein the metabolic disorder-related diseases include at least one of obesity, diabetes, dyslipidemia-related diseases, fatty liver disease, metabolic syndrome and non-alcoholic fatty liver disease.
